(19) Europäisches Patentamt
European Patent Office
Office européen des brevets

(11) EP 4 106 784 B1

(12) **FASCICULE DE BREVET EUROPEEN**

(45) Date de publication et mention de la délivrance du brevet:
**03.04.2024 Bulletin 2024/14**

(21) Numéro de dépôt: **21704808.1**

(22) Date de dépôt: **15.02.2021**

(51) Classification Internationale des Brevets (IPC):
***A61K 36/28*** *(2006.01)* ***A61K 8/9789*** *(2017.01)*
***A61P 17/00*** *(2006.01)* ***A61Q 19/00*** *(2006.01)*

(52) Classification Coopérative des Brevets (CPC):
**A61P 17/00; A61K 8/9789; A61K 36/28; A61Q 5/00; A61Q 19/00;** A61K 2800/84

(86) Numéro de dépôt international:
**PCT/EP2021/053648**

(87) Numéro de publication internationale:
**WO 2021/165205 (26.08.2021 Gazette 2021/34)**

(54) **LYSAT (LY) DE CELLULES DEDIFFERENCIEES DE LA PLANTE HELICHRYSUM STOECHAS ADMINISTRABLE PAR VOIE TOPIQUE POUR ÉLIMINER OU RÉDUIRE L'INFLAMMATION DE LA PEAU**

TOPISCH VERABREICHBARES LYSAT (LY) VON DEDIFFERENZIERTEN ZELLEN DER PFLANZE HELICHRYSUM STOECHAS ZUR BESEITIGUNG ODER MINDERUNG VON ENTZÜNDUNGEN DER HAUT

TOPICALLY ADMINISTRABLE LYSATE (LY) OF DEDIFFERENTIATED CELLS OF THE PLANT HELICHRYSUM STOECHAS FOR ELIMINATING OR REDUCING INFLAMMATION OF THE SKIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.02.2020 FR 2001548**

(43) Date de publication de la demande:
**28.12.2022 Bulletin 2022/52**

(60) Demande divisionnaire:
**24152338.0**

(73) Titulaire: **Société d'Exploitation de Produits pour les Industries Chimiques SEPPIC**
**75321 Paris cedex 07 (FR)**

(72) Inventeurs:
- **LE GELEBART, Erwan**
  **22260 QUEMPER-GUÉZENNEC (FR)**
- **BIZE, Cécile**
  **81100 CASTRES (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(56) Documents cités:
**FR-A1- 2 837 385** **FR-A1- 3 040 625**
**FR-A1- 3 079 144**

- **RECIO M C ET AL: "Anti-inflammatory activity of Helichrysum stoechas", PLANTA MEDICA, THIEME VERLAG, DE, vol. 57, no. suppl. 2, 1 janvier 1991 (1991-01-01), pages A56-A57, XP009113513, ISSN: 0032-0943, DOI: 10.1055/S-2006-960317**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Processed by Luminess, 75001 PARIS (FR)

## Description

La présente invention est relative à un lysat de cellules dédifférenciées de la plante *Helichrysum stoechas* administrable par voie topique pour apaiser la peau humaine et/ou le cuir chevelu, plus particulièrement les peaux sèches, réactives et/ou sensibles.

La peau constitue l'interface entre le milieu intérieur humain (ou organisme humain) et l'environnement extérieur. De ce fait, et avec la flore qui la recouvre et l'habite, la peau a notamment pour fonction d'assurer une mission de protection de l'organisme humain, en formant une véritable barrière qui est vitale. En particulier la peau permet de lutter contre la déshydratation en limitant la diffusion de l'eau hors de l'organisme.

En raison de sa position d'interface avec le milieu extérieur, la peau est soumise à des sollicitations quotidiennes nombreuses, telles que par exemple le contact avec des vêtements, les changements de températures, l'exposition aux rayonnements ultra-violets de la lumière du soleil, les changements de degrés d'hygrométrie, le contact avec certains produits chimiques irritants, le contact avec des produits chimiques considérés comme des agents polluants.

La peau est composée de couches de différents tissus :

- L'épiderme, composé de kératinocytes, est sa partie la plus externe, puis vient
- Le derme, qui est un tissu conjonctif composé principalement de fibroblastes et de la matrice extracellulaire, et
- L'hypoderme, constitué d'adipocytes, qui est la partie la plus profonde et la plus éloignée du milieu extérieur.

La peau assure diverses fonctions dans l'intérêt de l'ensemble du système qu'elle abrite parmi lesquelles on peut retenir :

- Une fonction de barrière mécanique pour garantir l'intégrité du milieu intérieur de l'organisme,
- Une fonction émonctorielle visant à sécréter de la sueur à base d'eau, de sels et de déchets acides,
- Une fonction de régulation de la température du corps, et contient bien d'autres mécanismes de régulation, comme par exemple son mécanisme d'adaptation et de protection face aux rayonnements ultra-violets (coloration pigmentaire adaptative par la production de la mélanine), comme par exemple un système de veille immunitaire par la présence de macrophages, de cellules dendritiques.

La peau humaine constitue aussi la première image offerte au regard d'autrui.

Par conséquent, l'amélioration de son aspect est un sujet de préoccupation constant pour les êtres humains. La peau est le reflet d'un état de bien-être, souvent associé à la jeunesse, et a contrario d'un état de fatigue et/ou de vieillissement. Il en résulte que la préservation, l'amélioration de l'état de la couche la plus extérieure de la peau, à savoir l'épiderme, constitue un centre d'intérêt majeur pour les recherches menées par les industries cosmétiques.

A la périphérie de l'épiderme, se trouve une couche cornée supérieure, nommée le *stratum corneum,* qui est la première couche de l'épiderme à subir les stress d'origine externe, comme les variations de conditions climatiques extérieures (température, hygrométrie, rayonnements ultra-violet, agents polluants) ou les sollicitations mécaniques. Concernant l'apaisement des peaux réactives et/ou sensibles, la peau sensible ou réactive est une condition qui touche beaucoup de personnes: environ 50% des personnes (60% des femmes, 40% des hommes) indiquent avoir la peau réactive.

Les peaux sensibles se définissent par une réactivité particulière de la peau.

Cette réactivité cutanée se traduit classiquement par la manifestation de signes d'inconfort, comme les rougeurs, en réponse à la mise en contact du sujet avec un élément déclenchant qui peut avoir diverses origines.

Elle a été définie il y a peu comme suit: "un syndrome défini par l'apparition de sensations désagréables (sensations de picotement, brûlure, douleur, prurit et de fourmillement) en réponse à des stimuli qui normalement ne devraient pas provoquer de telles sensations. Ces sensations ne peuvent pas être expliquées par des lésions liées à des maladies de peau. La peau peut sembler normale ou présenter en plus de l'érythème. La peau sensible peut affecter toutes les régions du corps, et en particulier le visage ».

Des facteurs environnementaux tels que l'exposition à des rayons aux ultraviolets ou infrarouges, et/ou à la pollution atmosphérique, et/ou à des variations brutales de température et/ou au vent, ou le mode de vie (habitudes alimentaires ou application de produits cosmétiques en surface de la peau), ou encore des facteurs physiologiques comme le stress, les hormones endogènes, ont été reconnus comme pouvant induire ou aggraver les symptômes des peaux sensibles. Deux raisons principales peuvent expliquer les symptômes des peaux sensibles: d'une part une augmentation de la perméabilité du *stratum corneum,* d'autre part une sécrétion excessive de certains neuromédiateurs par les terminaisons des nerfs superficiels et des cytokines pro-inflammatoires.

En effet, la peau sensible subit une cascade inflammatoire. La production excessive de cytokines pro-inflammatoires entretient l'inflammation des peaux sensibles et les manifestations visibles associées (rougeurs, tiraillements, démangeaisons, sensations inconfortables de chaleur).

Au sens de la présente invention, les peaux sensibles couvrent les peaux irritables et les peaux intolérantes.

Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyper-séborrhéique ou acnéique, voire même rosacéiforme, avec ou sans dartres.

Une peau irritable est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température, l'humidité ou la laine.

Partant de là, un problème qui se pose est de fournir un composé permettant d'apaiser la peau, de réduire son inflammation.

Une solution selon la présente invention est un lysat (Ly) de cellules dédifférenciées de la plante Helichrysum stoechas administrable par voie topique pour éliminer ou réduire l'inflammation de la peau et/ou du cuir chevelu.

Par « éliminer ou réduire l'inflammation de la peau humaine et/ou du cuir chevelu», on entend au sens de la présente invention le retour partiel ou total à l'état d'homéostasie des cellules enflammées.

Par « administrable par voie topique » on entend que le lysat est formulé pour être administrable par voie topique.

Le lysat (Ly) de cellules dédifférenciées de la plante Helichrysum stoechas sera le principe actif permettant d'éliminer l'inflammation de la peau et/ou du cuir chevelu.

Selon un aspect particulier, la présente invention concerne un lysat de cellules dédifférenciées de la plante *Helichrysum stoechas* administrable par voie topique pour pour une utilisation pour éliminer les signes inesthétiques et/ou symptomatiques liés à l'inflammation de la peau humaine et/ou du cuir chevelu comme par exemple les rougeurs, les tiraillements, les démangeaisons, les picotements.

De préférence, le lysat est issu de l'homogénéisation haute pression de la culture de cellules dédifférenciées de la plante Helichrysum stoechas.

Le procédé d'obtention du lysat de cellules dédifférenciées de la plante Helichrysum stoechas selon l'invention comprendra de préférence les étapes suivantes :

a) Préparation de l'échantillon stérile de plante Helichrysum stoechas,
b) Callogenèse
c) Mise en suspension
d) Sélection d'une suspension fine
e) Production de biomasse
f) Homogénéisation Haute Pression de la culture
g) Stabilisation du Lysat de culture de cellules dédifférenciées

A partir de l'étape e) les étapes a) b) c) d) n'ont plus besoin d'être reconduites car elle mène à la culture de cellules dédifférenciées utilisables à l'infini pour produire de la biomasse dans la mesure où les besoins physiologiques des cellules sont respectés.

Les étapes b) c) d) et e) doivent être réalisées en conditions stériles afin de maintenir la culture dans des conditions axéniques.

Les étapes sont détaillées comme suit :

a) Préparation de l'échantillon *d'Helichrysum stoechas* stérile

Prélever quelques feuilles d'Helichrysum stoechas, et découper ces feuilles en fragments de quelques millimètres. Stériliser les fragments de feuilles par immersion dans des bains successifs contenant :

- Un mélange d'éthanol 70% contenant un détergent tel que du Tween 80 afin d'améliorer l'efficacité de la stérilisation pendant une durée de 5 minutes, puis dans
- Une solution d'hypochlorite de sodium à 1% massique pendant une durée de 5 minutes

Puis rincer les fragments des feuilles ainsi stérilisées par immersion dans un bain d'eau distillée stérile à 3 reprises successives.

Cette étape a) peut également être réalisée en utilisant n'importe quelle autre partie de la plante (racines, tiges, méristèmes, fleurs...). La stérilisation peut aussi s'appliquer aux graines, sans la phase de découpe.

b) Callogenèse

On dépose des morceaux de feuilles stérilisées obtenues à l'étape précédente, sur le milieu de callogenèse

solide (dont la composition est décrite dans le tableau 1), qui est ensuite incubé pendant une durée de trois semaines dans une enceinte thermostatée à une température supérieure ou égale à 20 et inférieure ou égale à 25°C à la lumière ou à l'obscurité.

[Table 1]

| Composés | Concentration (mg/ L) |
|---|---|
| Glucose ou Saccharose | 30000 |
| Acide Naphtalène Acétique | 1 |
| 6-benzylaminopurine | 0.5 |
| Agar | 10 000 |
| $KNO_3$ | 2500 |
| $(NH_4)_2SO_4$ | 134 |
| $CaCl_2$, $2H_2O$ | 150 |
| $NaH_2PO_4$, $2H_2O$ | 150 |
| $MgSO_4$, $7H_2O$ | 250 |
| $MnSO_4$, $H_2O$ | 16,9 |
| $ZnSO_4$, $7H_2O$ | 8,6 |
| $H_3BO_3$ | 6,2 |
| KI | 0,83 |
| $Na_2MoO_4$, $2H_2O$ | 0,25 |
| $CuSO_4$, $5H_2O$ | 0,025 |
| $FeSO_4$, $7H_2O$ | 27,8 |
| $Na_2EDTA$, $2H_2O$ | 37,3 |
| Myo-inositol | 100 |
| Acide Nicotinique | 1 |
| Acide Panthotenique | 1 |
| Biotine | 0,01 |
| Pyridoxine | 1 |
| Thiamine | 1 |

composition du milieu de callogenèse

a) Mise en suspension des cellules dédifférenciées

Prélever le cal formé au cours de l'étape b) et le déposer dans un milieu liquide de mise en suspension (dont la composition est indiquée dans le tableau 2). Par « cal », on entend au sens de la présente invention un amas de cellules dédifférenciées.

Les morceaux de cal en suspension sont mis en culture dans enceinte thermostatée pendant une durée de 7 jours à 21 jours à une température supérieure ou égale à 20 et inférieure ou égale à 25°C à la lumière ou à l'obscurité, sous agitation mécanique de type orbitale, à une vitesse d'agitation comprise entre 25 tours/minute et 200 tours/minute (plus précisément à une vitesse d'agitation de 100 tours/minute pour diamètre orbital de 5 cm).

[Table 2]

| Composés | Concentration (mg/ L) |
|---|---|
| Glucose ou Saccharose | 30 000 |
| Acide Naphtalène Acétique | 1 |
| 6-benzylaminopurine | 0.5 |
| $KNO_3$ | 2500 |
| $(NH4)_2SO_4$ | 134 |
| $CaCl_2$, $2H_2O$ | 150 |
| $NaH_2PO_4$, $2H_2O$ | 150 |
| $MgSO_4$, $7H_2O$ | 250 |
| $MnSO_4$, $H_2O$ | 16,9 |
| $ZnSO_4$, $7H_2O$ | 8,6 |
| $H_3BO_3$ | 6,2 |
| KI | 0,83 |
| $Na_2MoO_4$, $2H_2O$ | 0,25 |
| $CuSO_4$,$5H_2O$ | 0,025 |
| $FeSO_4$, $7H_2O$ | 27,8 |
| $Na_2EDTA$, $2H_2O$ | 37,3 |
| Myo-inositol | 100 |
| Acide Nicotinique | 1 |
| Acide Panthotenique | 1 |
| Biotine | 0,01 |
| Pyridoxine | 1 |
| Thiamine | 1 |

composition du milieu de mise en suspension du cal formé à l'étape b).

a) Sélection d'une suspension fine

Prélever entre 1/5 et 1/3 du volume de culture contenant des cellules dédifférenciées en suspension fine obtenues à l'issue de l'étape c.

Déposer ce volume de suspension fine dans un volume de milieu de mise en suspension frais, trois fois supérieur au volume de suspension fine.

Cette nouvelle suspension est mise en culture dans une enceinte thermostatée pendant une durée de 7 à 21 jours (la fin de la période de culture est dictée par l'observation de la carence en nutriments) à une température supérieure ou égale à 20 et inférieure ou égale à 25°C à la lumière, sous agitation mécanique de type orbitale, à une vitesse de rotation comprise entre 25 tours/minute et 200 tours/minute (plus précisément à une vitesse de rotation de 100 tours/minute pour un diamètre orbital de 5 cm). Par « carence en nutriment », on entend au sens de la présente invention l'observation d'une teneur inférieure à 5% de la quantité de nutriment initialement introduite dans le milieu de culture.

b) Production de biomasse comprenant des cellules dédifférenciées et le milieu de culture

Prélever un volume de la suspension fine obtenue au cours de l'étape d), et le déposer dans un volume de milieu de mise en suspension frais, tel que décrit dans le tableau 2, 3 fois supérieur au volume prélevé de suspension fine obtenue à l'issu de l'étape d).

Cette nouvelle suspension est mise en culture dans une enceinte thermostatée pendant une durée de 7 à 21 jours (la fin de la période de culture est dictée par la carence en nutriments), à une température supérieure ou égale à 20 et inférieure ou égale à 25°C, à la lumière ou à l'obscurité, sous agitation mécanique de type orbitale, à une vitesse de rotation comprise entre 25 tours/minute et 200 tours/minute (plus précisément à 100 tours/minute pour diamètre orbital de 5 cm). Cette étape peut être réalisée, jusqu'à des volumes inférieurs ou égaux à 5 litres dans des Erlenmeyers, puis pour des volumes supérieurs à 5 litres et inférieurs ou égaux à 1000 Litres dans des bioréacteurs (type « réacteur à vagues » ou « wave », « réacteur à cuve agitée » ou « stired tank » ou erlenmeyer) (cette liste n'étant pas exhaustive).

a) Homogénéisation à Haute Pression de la culture : obtention du lysat de culture de cellules dédifférenciées.

Une fraction du volume de culture obtenu à l'issu de l'étape e) est prélevée pour être lysée en utilisant un homogénéisateur à haute pression, dont la pression peut être fixée à une valeur supérieur ou égale à 100 et inférieure ou égale à 1000 bars (de préférence pour la présente invention, à une pression égale à 500 bars). Les cellules dédifférenciées présentes dans la culture passée dans l'homogénéisateur haute pression sont lysées, et leur contenu est libéré dans le milieu de culture. L'efficacité d'homogénéisation (se traduisant par l'observation de débris cellulaires témoins de la lyse des cellules dédifférenciées) est facilement vérifiable par observation microscopique de la suspension.

b) Stabilisation du Lysat de culture de cellules dédifférenciées

Le lysat de culture de cellules dédifférenciées collecté à l'issu de l'étape f) est acidifié, par l'ajout d'un acide minéral ou d'un acide organique, pour atteindre une valeur de pH supérieure ou égale à 4 et inférieure ou égale à 4,5 afin de stabiliser sa couleur. Ce lysat, contient des morceaux de cellules insolubles dans le milieu et qui sont susceptibles de sédimenter, et de constituer un frein à la commercialisation du produit. L'addition d'un agent -épaississant et/ou gélifiant, dans une proportion supérieure ou égale à 0,1% massique et inférieure ou égale à 2% (plus préférentiellement un mélange de gomme de xanthane et d'acacia, dans des proportions massiques de 40-45% et 48-55% et en quantité de 0,8% massique, comme le mélange de gomme de xanthane et de gomme d'acacia commercialisé sous le nom de marque Solagum™AX) permet d'obtenir une suspension ne sédimentant pas dans le temps.

Un lysat de cellules dédifférenciées peut être stabilisé d'un point de vue microbiologique et physicochimique par différents moyens :

- Par pasteurisation (exemple: pasteurisation UHT),
- Par modification du pH (Exemple: acidification pH<5),
- Par addition d'un agent de réduction (Exemple: glycérine) de la disponibilité en eau,
- Par addition de conservateurs (Exemple: benzoate de sodium, sorbate de potassium), Dans notre cas, nous préférons stabiliser notre lysat par l'addition d'un mélange de glycérine, de sorbate de potassium et de benzoate de sodium.

Les proportions des différents ingrédients sont les suivantes, pour 100% massique :

- 60% à 70% massique de lysat HHP de la culture de cellules dédifférenciées d'Helichrysum stoechas, obtenu à l'étape f (95% à 99,5 % massique d'eau et 0,5% à 5% massique de matière sèche).
- 30% à 40% massique de glycérol
- 0,1% à 2% massique de Solagum™ AX (mélange de gomme d'acacia et xanthane)
- 0,1% à 2% de sorbate de potassium
- 0,1% à 2% de benzoate de sodium

Le lysat de culture de cellules dédifférenciées stabilisé peut être stocké ou être directement incorporé à une formulation cosmétique à hauteur de 0,1% à 3% massique (préférentiellement 1% massique).

Tout type d'agent épaississant peut remplir la fonction d'agent stabilisant, par exemple Nous utilisons un solvant de nature polaire, de préférence le glycérol, mais d'autres polyols pourraient être utilisés

Notons qu'il existe bien des différences entre la plante entière et les cellules dédifférenciées. Pour montrer ces différences, nous avons effectué une comparaison. La comparaison entre la plante entière et les cellules dédifférenciées a pu être faite grâce à la comparaison d'extraits éthanoliques analysés en chromatographie liquide à haute pression (ou « HPLC »). Les échantillons sont analysés sur une colonne HPLC Kinetex C18 150*4.6 mm avec un débit de 0.8 mL/min. Les solvants sont les suivants :

- A : isopropanol à 0,05% TFA,
- B : acetonitrile à 0,05% TFA,

- C : eau à 0,05% TFA.

Le gradient est le suivant :

| t (min) | % A | % B | % C |
|---|---|---|---|
| 0 | 0 | 2 | 98 |
| 5 | 0 | 2 | 98 |
| 20 | 0 | 100 | 0 |
| 25 | 0 | 100 | 0 |
| 35 | 80 | 20 | 0 |
| 40 | 80 | 20 | 0 |
| 45 | 0 | 100 | 0 |
| 50 | 0 | 2 | 98 |

Cette analyse a été réalisée avec une détection de type CAD (Charged Aérosol Détection). Les résultats sont obtenus d'un chromatogramme d'un extrait éthanolique de parties aériennes d'*Helichrysum stoechas* en détection CAD selon la méthode HPLC C18_screening et d'un chromatogramme d'un extrait éthanolique de cellules dédifférenciées d'*Helichrysum stoechas* en détection CAD selon la méthode HPLC C18_screening.
Les résultats obtenus montrent bien une différence entre les deux chromatogrammes. Le chromatogramme de l'extrait de cellules est beaucoup plus riche dans la zone entre les temps de rétention compris entre 25 minutes et 40 minutes, ce qui correspond aux temps d'élution des molécules les plus apolaires.
La présente invention a également pour objet une composition à usage topique (C1) se présentant sous la forme d'un gel et comprenant pour 100% de sa masse :

- De 95% à 99,5% massique, de préférence de 97% à 99,5% massique, encore plus préférentiellement de 97,5% à 99,5% massique du Lysat (Ly) tel que défini précédemment et obtenu de préférence par la mise en oeuvre d'un procédé comprenant les étapes a) à f) telles que décrites ci-dessus,
- De 0,5% à 5% massique, de préférence de 0,5% à 3% massique, encore plus préférentiellement de 0,5% à 2,5% massique d'au moins un agent épaississant et/ou gélifiant.

De préférence les agents gélifiants et/ou épaississants sont choisis parmi les polysaccharides, la cellulose et les dérivés de la cellulose, les amidons, et les polymères de type polyélectrolytes, linéaires ou branchés ou réticulés.
Cette composition (C1) pourra être administrable par voie topique pour éliminer ou réduire l'inflammation de la peau et/ou du cuir chevelu. Par « administrable par voie topique », on entend que la composition est formulée pour être administrable par voir topique.
Selon un aspect encore plus particulier, la présente invention concerne une composition (C1) administrable par voie topique pour éliminer ou réduire les signes inesthétiques et/ou symptomatiques liés à l'inflammation de la peau humaine sensible et/ou du cuir chevelu comme par exemple les rougeurs, les tiraillements, les démangeaisons, les picotements. Dans le cadre de la présente invention, on désigne par « gel » une composition chimique se présentant initialement à l'état liquide qui, après ajout de substances gélifiantes et/ou épaississantes, est transformé en un état structuré, qui ne coule pas. Un gel tel que défini, est considéré comme un état intermédiaire entre l'état solide et l'état liquide, et est constitué par un réseau tridimensionnel au sein du liquide étant le résultat de liaisons chimiques ou physiques.
Par « agent gélifiant », on entend au sens de la présente invention un composé chimique ou un mélange de composés chimiques, qui transforme un milieu liquide en un gel.
Par « agent épaississant », on entend au sens de la présente invention un composé chimique ou un mélange de composés chimiques, qui augmente la viscosité du milieu dans lequel il est introduit.
L'expression "à usage topique" utilisée dans la définition de la composition ($C_1$) telle que décrite ci-dessus, signifie que ladite composition sera formulée pour permettre son application sur la peau, les cheveux, le cuir chevelu, les ongles, les lèvres, les muqueuses, les cils, les sourcils, qu'il s'agisse d'une application directe dans le cas d'une formulation cosmétique ou d'une application indirecte par exemple dans le cas d'un produit de soin corporel sous forme de lingette en textile ou en papier ou de produits sanitaires destinés à être en contact avec la peau, les cheveux, le cuir chevelu, les ongles, les lèvres, les muqueuses, les cils, les sourcils.

Dans le cadre de la présente invention, par polysaccharides, on désigne les polymères de saccharides. La définition IUPAC des saccharides désigne des oses, des composés d'oses proprement dits et leurs dérivés obtenus soit par réduction d'un groupement carbonyle, soit par oxydation d'une ou plusieurs fonctions hydroxyles, soit par le remplacement d'une ou plusieurs fonctions hydroxyles par un atome d'hydrogène, par un groupement amine, une fonction phosphate, une fonction sulfate. Les polysaccharides les plus couramment utilisés pour la préparation de compositions industrielles, alimentaires, cosmétiques ou pharmaceutiques, sont majoritairement constitués d'oses, tels que le glucose, le galactose, le mannose ou de dérivés d'oses pour lesquels la fonction hydroxyle du carbone terminal a été oxydée en fonction carboxyle. On peut distinguer parmi les polysaccharides deux groupes distincts: les polysaccharides constitués uniquement d'oses (ou poly-oses) et les polysaccharides constitués de dérivés d'oses.

Selon un aspect particulier, les agents gélifiants et/ou épaississants présents dans la composition aqueuse (C1) objet de la présente invention, sont choisis parmi les polysaccharides constitués uniquement d'oses (ou poly-oses).

Parmi les polysaccharides composés uniquement d'oses, on peut distinguer les glucanes, qui sont des homopolymères de glucose très abondants dans la nature, les glucomannoglycanes, les xyloglycanes et les galactomannanes, qui sont des polymères dont la chaîne principale est constituée d'unités de D-mannose, reliées entre elles en $\beta$-1,4, et sur laquelle des unités de D-galactose sont greffées latéralement par des liaisons $\alpha$-1,6.

Les galactomannanes sont présents dans plusieurs espèces végétales, et plus particulièrement dans les espèces légumineuses dans lesquelles ils constituent l'albumen des graines. Selon leur origine végétale, le degré de substitution (DS) des unités de D-galactose sur la chaîne principale de D-mannose des galactomannanes, varie entre 0 et 1:

- les galactomannanes provenant de la gomme de cassia présentent un degré de substitution (DS) d'environ 1/5, signifiant le greffage latéral d'une unité de D-galactose toutes les 5 unités de D-mannose présentes sur la chaîne principale du polysaccharide.
- Les galactomannanes provenant de la gomme de caroube présentent un degré de substitution (DS) d'environ 1/4, signifiant le greffage latéral d'une unité de D-galactose toutes les 4 unités de D-mannose présentes sur la chaîne principale du polysaccharide
- Les galactomannanes provenant de la gomme de tara présentent un degré de substitution (DS) d'environ 1/3, signifiant le greffage latéral d'une unité de D-galactose toutes les 3 unités de D-mannose présentes sur la chaîne principale du polysaccharide.
- Les galactomannanes provenant de la gomme de guar présentent un degré de substitution (DS) d'environ 1/2, signifiant le greffage latéral d'une unité de D-galactose toutes les 2 unités de D-mannose présentes sur la chaîne principale du polysaccharide
- Les galactomannanes provenant de la gomme de fenugrec présentent un degré de substitution (DS) d'environ 1/1, signifiant le greffage latéral d'une unité de D-galactose pour quasiment toutes les unités de D-mannose présentes sur la chaîne principale du polysaccharide.

Selon un aspect plus particulier, les agents gélifiants et/ou épaississants présents dans la composition aqueuse (C1) objet de la présente invention, sont choisis parmi les polysaccharides constitués uniquement d'oses (ou poly-oses) compris dans le groupe constitué du galactomannane provenant de la gomme de Tara, du galactomannane provenant de la gomme de guar et du galactomannane provenant de la gomme de caroube.

Selon un autre aspect particulier, les agents gélifiants et/ou épaississants présents dans la composition aqueuse ($C_1$) objet de la présente invention, sont choisis parmi les polysaccharides constitués de dérivés d'oses. Parmi les polysaccharides constitués de dérivés d'oses, on peut distinguer:

- Les galactanes sulfatés, qui sont des polymères de galactose pouvant avoir des groupements esters-sulfate appendus, représentés notamment par les polyosides algaux comme les carraghénanes et l'agar ;
- Les uronanes, qui sont les polymères d'acides uroniques comme les algines et les pectines ;
- Les hétéropolymères d'oses et d'acides uroniques: souvent de composition complexe, ces polymères se trouvent notamment dans les exsudats de sève (comme par exemple l'exsudat de la gomme arabique et l'exsudat de la gomme de karaya) mais ils sont produits aussi par des microorganismes, comme par exemple la gomme xanthane et la gomme gellane ;
- Les glucosaminoglycanes qui sont des polyosides formés à partir d'un glucose dérivé par remplacement de son hydroxyle sur C-2 par une amine (appelé 2-amino-2-désoxy-D-glucose ou, plus simplement, glucosamine). La fonction amine peut être d'ailleurs acétylée. Parmi les hydrocolloïdes dans cette classe on trouve le chitosane, formé uniquement de motifs glucosamine, et l'hyaluronane, dont l'unité de répétition est un dimère de glucosamine et d'acide glucuronique.

La gomme xanthane (Gx) est devenue depuis quelques décennies le polyoside microbien le plus utilisé dans

l'industrie. Le xanthane est un polysaccharide synthétisé par des bactéries du genre *Xanthomonas* et, commercialement, on utilise uniquement l'espèce *X. campestris.* La chaîne principale de la (Gx) est identique à celle de la cellulose, c'est-à-dire qu'elle est formée d'unités $\beta$-$_D$-glucose reliées par les carbones 1 et 4. On compte un triholoside branché toutes les deux unités de glucose dans la chaîne principale, de façon alternative régulière ; chaque branchement consistant en un triholoside composé de deux mannoses et d'un acide glucuronique, du type: $\beta$-$_D$-Manp-(1 $\rightarrow$ 4)-$\beta$-$_D$-GlcAp-(1 --> 2)-$\alpha$-$_D$-Manp-(1 --> 3) [I. Capron et al. *"About the native and renaturated conformation of xanthan exopolysaccharide".* 1997). La gomme xanthane (GX) est disponible sous forme d'un sel de sodium, de potassium ou de calcium.

La gomme d'acacia est un polysaccharide complexe branché dont la chaîne principale consiste en des unités de $\beta$-$_D$-galactose reliées entre elles par les carbones 1 et 3. Les chaînes branchées à la chaîne principale sont constituées d'unités de $\beta$-$_D$-galactose reliées entre elles par les carbones 1 et 6, portant également des unités de $\alpha$-arabinose, et dans de moindres proportions des unités $\beta$-glucoronosyle. A la fois la chaîne principale et les chaînes pendantes contiennent des unités $\alpha$-L-arabinosyle, $\alpha$-L-rhamnopyranosyle, $\beta$-D-glucuronopyranosyle et 4-O-méthyl-$\beta$-D-glucuronopyranosyle.

Selon un aspect plus particulier, les agents gélifiants et/ou épaississants présents dans la composition aqueuse ($C_1$) objet de la présente invention sont des polysaccharides constitués de dérivés d'oses choisis parmi les éléments du groupe constitué par les carraghénanes, l'agar, les algines, les pectines, l'exsudat de la gomme arabique, l'exsudat de la gomme de karaya, la gomme de xanthane, la gomme de gellane, le chitosane et l'hyaluronane, et/ou leurs mélanges. Selon un aspect encore plus particulier, les agents gélifiants et/ou épaississants présents dans la composition aqueuse ($C_1$) objet de la présente invention sont des polysaccharides constitués de dérivés d'oses choisis parmi les éléments du groupe constitué par l'exsudat de la gomme arabique, l'exsudat de la gomme de karaya, la gomme de xanthane et/ou leurs mélanges. Selon un aspect encore plus particulier, les agents gélifiants et/ou épaississants présents dans la composition aqueuse ($C_1$) objet de la présente invention sont des polysaccharides constitués de dérivés d'oses choisis parmi les éléments du groupe constitué par l'exsudat de la gomme arabique, la gomme de xanthane, le mélange de gomme de xanthane (Gx) et d'exsudat de la gomme arabique ($G_A$) utilisé dans un ratio massique entre la gomme de xanthane (Gx) et la gomme d'exsudat d'acacia ($G_A$) est supérieur ou égal à 1/3 et inférieur ou égal à 3/1, notamment commercialisé par la société SEPPIC sous le nom de marque SOLAGUM™AX. Selon un aspect particulier, les agents gélifiants et/ou épaississants présents dans la composition aqueuse ($C_1$) objet de la présente invention, sont choisis parmi la cellulose et les dérivés de la cellulose.

Dans le cadre de la présente invention, par « cellulose » on désigne un polysaccharide qui est constitué par une chaîne linéaire de molécules de D-Glucose, dont la masse moléculaire moyenne est d'au moins 10.000$gmol^{-1}$, plus particulièrement d'au moins 15.000$gmol^{-1}$, plus particulièrement d'au moins 17.000$gmol^{-1}$, encore plus particulièrement d'au moins 20.000$gmol^{-1}$, encore plus particulièrement d'au moins 25.000$gmol^{-1}$.

Selon un aspect plus particulier, les agents gélifiants et/ou épaississants présents dans la composition aqueuse ($C_1$) objet de la présente invention, sont choisis parmi les dérivés de cellulose.

Dans le cadre de la présente invention, par « dérivés de cellulose » on désigne les éléments du groupe constitué par l'hydroxy-éthyl cellulose, la méthyl cellulose, l'éthyl cellulose, la méthyl hydroxy éthyl cellulose, la méthyl hydroxy propyl cellulose, l' hydroxy propyl cellulose, le sel de sodium de la carboxy méthyl cellulose, le dihydroxy propyl éther de cellulose.

Dans le cadre de la présente invention, par «amidon » on désigne un mélange d'amylose et d'amylopectine, et plus particulièrement les éléments du groupe constitué par l'amidon de maïs, l'amidon de blé, l'amidon de patate et l'amidon de manioc.

Selon un aspect particulier, par «polymères de type polyélectrolytes, linéaires ou branchés ou réticulés », on désigne au sens de la présente invention:

- les copolymères anioniques synthétiques réticulés à base d'acide méthacrylique ou d'acide acrylique, ou d'esters de l'acide méthacrylique ou de l'acide acrylique, optionnellement hydrophiquement modifiés, préparés par polymérisation en émulsion directe. Ces copolymères anioniques synthétiques sont respectivement connus de l'homme du métier sous les appellations "Alcaline Swellable Emulsion" (ou "ASE") et "Hydrophobically Alcaline Swellable Emulsion" (ou "HASE"). Des agents épaississants de type "HASE" sont décrits dans la demande internationale publiée sous le numéro WO 02/34793 A2 ;
- Les polyélectrolytes anioniques synthétiques, réticulés ou branchés, qui sont des homopolymères ou des copolymères réticulés et/ou branchés de monomères insaturés hydrosolubles, comme l'acide acrylique et/ou ses dérivés, l'acide méthacrylique et/ou ses dérivés, l'acrylamide et/ou ses dérivés, l'acide 2-acrylamido-2-méthylpropanesulfonique et/ou de ses sels, la N-vinyl pyrrolidone, l'alcool vinylique et/ou ses dérivés. Ces polyélectrolytes anioniques synthétiques, réticulés ou branchés, se présentent sous la forme de latex inverses, obtenus par polymérisation radicalaire en émulsion inverse, ou sous la forme de poudres, obtenues par polymérisation précipitante ou par atomisation de latex inverses.

Selon un aspect particulier, les agents gélifiants et/ou épaississants présents dans la composition aqueuse ($C_1$) objet de la présente invention, sont choisis parmi les polyélectrolytes linéaires ou branchés ou réticulés, issus de la polymérisation radicalaire d'au moins un monomère sélectionné parmi les éléments du groupe constitué par l'acide acrylique et/ou de son sel de sodium, de l'acide méthacrylique et/ou de son sel de sodium, de l'acrylate de 2-hydroxy éthyle, du méthacrylate de 2-hydroxyéthyle, de l'acrylamide, du N,N-diméthyl acrylamide, de la N-isopropyl acrylamide, l'acide 2-acrylamido-2-méthylpropanesulfonique et/ou son sel de sodium ou de potassium, la N-vinyl pyrrolidone, au moins un monomère de formule (I) :

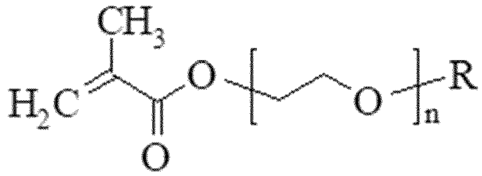

(I)

dans laquelle R représente un radical alkyle linéaire ou ramifié comportant de huit à vingt atomes de carbone et n représente un nombre entier supérieur ou égal à zéro et inférieur ou égal à vingt. Ladite polymérisation radicalaire est conduite en présence d'un agent réticulant choisi parmi les monomères polyéthyléniques comprenant au moins deux fonctions éthyléniques, et plus particulièrement choisi parmi les éléments du groupe constitué par le diméthacrylate d'éthylèneglycol, le tétraallyloxyéthane, le diacrylate d'éthylèneglycol, le diallyl urée, le triallyl amine, le triméthylol propanetriacrylate ou le méthylène-bis(acrylamide) ou un mélange de ces composés.

Selon un aspect particulier, les agents gélifiants et/ou épaississants présents dans la composition aqueuse ($C_1$) objet de la présente invention, sont choisis parmi les éléments du groupe constitué par:

- L'homopolymère de l'acide acrylique partiellement ou totalement salifié, réticulé au triallyl amine et/ou au triméthylol propanetriacrylate et/ou au méthylène-bis(acrylamide),
- L'homopolymère du sel de sodium de l'acide 2-acrylamido 2-méthyl propanesulfonique, réticulé au triallyl amine et/ou au triméthylol propanetriacrylate et/ou au méthylène-bis(acrylamide),
- Le copolymère du sel de sodium de l'acide 2-acrylamido 2-méthyl propanesulfonique et de l'acide acrylique partiellement ou totalement salifié, réticulé au triallyl amine et/ou au triméthylol propanetriacrylate et/ou au méthylène-bis(acrylamide),
- Le copolymère du sel de sodium de l'acide 2-acrylamido 2-méthyl propanesulfonique et de l'acrylate de 2-hydroxy éthyle, réticulé au triallyl amine et/ou au triméthylol propanetriacrylate et/ou au méthylène-bis(acrylamide),
- Le copolymère du sel de sodium de l'acide 2-acrylamido 2-méthyl propanesulfonique et de l'acrylamide, réticulé au triallyl amine et/ou au triméthylol propanetriacrylate et/ou au méthylène-bis(acrylamide),
- Le terpolymère du sel de sodium de l'acide 2-acrylamido 2-méthyl propanesulfonique, de l'acrylamide et l'acide acrylique partiellement ou totalement salifié, réticulé au triallyl amine et/ou au triméthylol propanetriacrylate et/ou au méthylène-bis(acrylamide),
- Le terpolymère du sel de sodium de l'acide 2-acrylamido 2-méthyl propanesulfonique, du N,N-diméthylacrylamide, et l'acide acrylique partiellement ou totalement salifié, réticulé au triallyl amine et/ou au triméthylol propanetriacrylate et/ou au méthylène-bis(acrylamide),
- un terpolymère réticulé par le trimethylolpropanetriacrylate et/ou le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 60% et inférieure ou égale à 80%, du N,N diméthylacrylamide dans une proportion molaire supérieure ou égale à 15% et inférieure ou égale à 39,5% et du méthacrylate de lauryle tétraéthoxylé dans une proportion molaire supérieure ou égale à 0,5% et inférieure ou égale à 5%,
- un tétrapolymère réticulé par le trimethylolpropanetriacrylate et/ou le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 60% et inférieure ou égale à 80%, du N,N diméthylacrylamide dans une proportion molaire supérieure ou égale à 15% et inférieure ou égale à 39 %, du méthacrylate de lauroyle dans une proportion molaire supérieure ou égale à 0,5% et inférieure ou égale à 2 ,5%, et du méthacrylate de stéaroyle dans une proportion molaire supérieure ou égale à 0,5% et inférieure ou égale à 2,5%.

Selon un aspect plus particulier, les agents gélifiants et/ou épaississants présents dans la composition aqueuse ($C_1$) objet de la présente invention, sont choisis parmi les éléments du groupe constitué par la gomme de xanthane,

l'exsudat de la gomme d'acacia, le mélange de gomme de xanthane (Gx) et d'exsudat de la gomme arabique ($G_A$) dans un ratio massique entre la gomme de xanthane (Gx) et la gomme d'exsudat d'acacia ($G_A$) est supérieur ou égal à 1/3 et inférieur ou égal à 3/1, le copolymère du sel de sodium de l'acide 2-acrylamido 2-méthyl propanesulfonique et de l'acrylate de 2-hydroxy éthyle, réticulé au triallyl amine et/ou au triméthylol propanetriacrylate et/ou au méthylène-bis(acrylamide), le copolymère du sel de sodium de l'acide 2-acrylamido-2-méthyl propanesulfonique et de l'acrylamide, réticulé au triallyl amine et/ou au triméthylol propanetriacrylate et/ou au méthylène-bis(acrylamide), le terpolymère réticulé par le trimethylolpropanetriacrylate et/ou le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 60% et inférieure ou égale à 80%, du N,N diméthylacrylamide dans une proportion molaire supérieure ou égale à 15% et inférieure ou égale à 39,5% et du méthacrylate de lauryle tétraéthoxylé dans une proportion molaire supérieure ou égale à 0,5% et inférieure ou égale à 5%.

La composition à usage topique ($C_1$) telle que définie précédemment, peut comprendre en outre, un ou plusieurs composés auxiliaires choisis parmi les solvants et cosolvants, les agents d'amélioration de la pénétration cutanée.

La présente invention a également pour objet une composition à usage topique (C'1) se présentant sous la forme d'un gel et comprenant pour 100% de sa masse :

- De 50% à 80% massique, de préférence de 50 à 75% massique, et encore plus préférentiellement de 60 à 70% massique du Lyzat (Ly) tel que défini précédemment,
- De 0,1% à 5% massique, de préférence de 0,5% à 3% massique d'au moins un agent épaississant et/ou gélifiant, et
- De 15% à 49,9% massique, de préférence de 22% à 49,5% massique, et encore plus préférentiellement de 27% à 39,5% massique d'au moins un solvant.

De préférence, le solvant est choisi parmi les éléments du groupe constitué par :

- les composés de formule (Ia) :

HO-[CH2-CH(OH)-CH2-O]n-H (Ia)

dans laquelle n représente un nombre entier supérieur ou égal à un et inférieur ou égal à quinze, plus particulièrement supérieur ou égal à un et inférieur ou égal à 10, plus particulièrement supérieur ou égal à un et inférieur ou égal à 6, plus particulièrement supérieur ou égal à un et inférieur ou égal à 4, plus particulièrement égal à 1 ou 2 ou 3 ou 4 ;

- les composés de formule (Ib) :

Ra1-C(Rb1)(OH)-C(OH)(Rc1)(Rd1) (Ib),

dans laquelle chacun des radicaux Ra1, Rb1, Rc1 et Rd1 représentent indépendamment les uns des autres, un atome d'hydrogène ou radical aliphatique saturé comportant de un à cinq atomes de carbone, soit par la formule (Ib1) :

Ra1-C(Rb1)(OH)-[C(Re1)(Rf1)]t-C(OH)(Rc1)(Rd1) (Ib1),

dans laquelle t est égal à un, deux ou trois, chacun des radicaux Ra1, Rb1, Rc1, Rd1, Re1 et Rf1 représentent indépendamment les uns des autres, un atome d'hydrogène ou radical aliphatique saturé comportant de un à cinq atomes de carbone, étant entendu que l'un au moins des radicaux Ra1 ou Rb1 et/ou l'un au moins des radicaux Rc1 ou Rd1 ne représentent pas un atome d'hydrogène.

Encore plus préférentiellement le solvant sera du glycérol.

Cette composition (C'1) pourra être administrable par voie topique pour éliminer ou réduire l'inflammation de la peau et/ou du cuir chevelu.

Selon un aspect encore plus particulier, la présente invention concerne une composition (C'1) administrable par voie topique pour éliminer ou réduire les signes inesthétiques et/ou symptomatiques liés à l'inflammation de la peau humaine sensible et/ou du cuir chevelu comme par exemple les rougeurs, les tiraillements, les démangeaisons, les picotements.

Les compositions à usage topique ($C_1$) et ($C'_1$) telles que définies précédemment, peuvent comprendre en outre, un ou plusieurs co-solvants, et agents d'amélioration de la pénétration cutanée.

Comme exemples de co-solvants éventuellement présents dans les compositions à usage topique ($C_1$) et ($C'_1$) objet de la présente invention, on peut citer l'eau, les solvants organiques par exemple le glycérol, le diglycérol, les oligomères du glycérol, l'éthylène glycol, le propylène glycol, le butylène glycol, l'hexylène glycol, le diéthylène glycol, le xylitol, l'érythritol, le sorbitol, les alcools hydrosolubles tels que l'éthanol, l'isopropanol ou le butanol, les mélanges

d'eau et desdits solvants organiques, le propylène carbonate, l'acétate d'éthyle, l'alcool benzylique.

Comme exemples d'agents d'amélioration de la pénétration cutanée éventuellement présents dans les compositions à usage topique ($C_1$) et ($C'_1$) objet de la présente invention, on peut citer les glycols éthers comme par exemple l'éthylène glycol mono méthyl éther, l'éthylène glycol mono éthyl éther, l'éthylène glycol mono propyl éther, l'éthylène glycol mono isopropyl éther, l'éthylène glycol mono butyl éther, l'éthylene glycol mono phényl éther, l'éthylène glycol mono benzyl éther, le diéthylène glycol mono méthyl éther, le diéthylène glycol mono éthyl éther et le diéthylène glycol mono-n-butyl éther, le diéthylène glycol mono éthyléther (ou Transcutol-P), les acides gras comme l'acide oléique, les esters de glycérol d'acide gras comme par exemple le béhénate de glycérol, le palmitostéarate de glycérol, le béhénoyl macroglycérides, le polyoxyéthylène-2-stéaryl éther, le polyoxyéthylène-2-oléyl éthers, des terpènes comme par exemple le D-Limonène, des huiles essentielles comme par exemple l'huile essentielle d'eucalyptus.

La présente invention a également pour objet une composition à usage topique (C2) se présentant sous la forme d'une émulsion de type eau-dans-huile ou d'une émulsion de type huile-dans-eau, comprenant le lysat (Ly) tel que défini précédemment, et obtenu de préférence par la mise en oeuvre d'un procédé comprenant les étapes a) à f) telles que décrites ci-dessus.

On distingue les émulsions huile-dans-eau (H/E) dont la phase continue est constituée par une phase hydrophile, généralement une phase aqueuse, et la phase dispersée par une phase grasse lipophile, et les émulsions eau-dans-huile (E/H) dont la phase continue est constituée par une phase grasse lipophile et la phase dispersée par une phase hydrophile, généralement une phase aqueuse.

Cette composition (C2) pourra également être administrable par voie topique pour éliminer ou réduire l'inflammation de la peau et/ou du cuir chevelu.

Selon un aspect encore plus particulier, la présente invention concerne une composition (C2) administrable par voie topique pour éliminer ou réduire les signes inesthétiques et/ou symptomatiques liés à l'inflammation de la peau humaine sensible et/ou du cuir chevelu comme par exemple les rougeurs, les tiraillements, les démangeaisons, les picotements. Notons que les picotements et/ou les fourmillements et/ou les démangeaisons et/ou les échauffements et/ou les rougeurs et/ou l'inconfort cutané et/ou les tiraillements accompagnent les pathologies cutanées comme l'urticaire, les dermatites eczémateuses, la rosacée, le psoriasis, l'herpès, les photodermatoses, la dermatite atopique, la dermatite de contact, le lichen, les prurigos, les maladies prurigineuses, les fibroses, les troubles de la maturation du collagène, la sclérodermie, l'eczéma.

Parmi les émulsions de type eau-dans-huile , la présente invention a pour objet particulier une composition à usage topique (F) se présentant sous la forme d'une émulsion eau-dans-huile et comprenant pour 100% de sa masse :

- de 60% à 90% massique de la composition à usage topique (C1) ou (C'1) telle que définie ci-dessus,
- de 10 % à 40 % massique d'une phase grasse ($A_2$) comprenant i) au moins une huile, et optionnellement au moins une cire et ii) un système émulsionnant comprenant au moins un agent tensioactif émulsionnant ($S_1$).

De préférence, l'agent tensioactif émulsionnant ($S_1$) sera choisi parmi les éléments du groupe constitué par les compositions d'alkylpolyglycosides, les compositions d'alkylpolyglycosides et d'alcools gras, les esters de polyglycérols, les esters de polyglycérols alcoxylés, les polyhydroxystéarates de polyglycols, les polyhydroxystéarates de polyglycérols, les polyhydroxystéarates de polyglycérols alcoxylés.

Dans la formulation (F) à usage topique objet de la présente invention, on désigne par « huile » un composé et/ou un mélange de composés insoluble dans l'eau, et liquide à 25°C, et plus particulièrement :

- Les alcanes linéaires comportant de onze à dix-neuf atomes de carbone ;
- Les alcanes ramifiés, comportant de sept à quarante atomes de carbone, comme l'isododécane, l'isopentadécane, l'isohexadécane, l'isoheptadécane, l'isooctadécane, l'isononadécane ou l'isoeicosane), ou des mélanges de certains d'entre eux comme ceux cités ci-après et identifiés par leur nom INCI : $C_{7-8}$ isoparaffin, $C_{8-9}$ isoparaffin, $C_{9-11}$ isoparaffin, $C_{9-12}$ isoparaffin, $C_{9-13}$ isoparaffin, $C_{9-14}$ isoparaffin, $C_{9-16}$ isoparaffin, $C_{10-11}$ isoparaffin, $C_{10-12}$ isoparaffin, $C_{10-13}$ isoparaffin, $C_{11-12}$ isoparaffin, $C_{11-13}$ isoparaffin, $C_{11-14}$ isoparaffin, $C_{12-14}$ isoparaffin, $C_{12-20}$ isoparaffin, $C_{13-14}$ isoparaffin, $C_{13-16}$ isoparaffin ;
- Les cyclo-alcanes optionnellement substitués par un ou plusieurs radicaux alkyles linéaires ou ramifiés ;
- Les huiles blanches minérales, comme celles commercialisées sous les noms suivants : Marcol™52, Marcol™82, Drakeol™6VR, Eolane™130, Eolane™150 ;
- L'hémisqualane (ou 2,6,10-trimethyl- dodécane ; numéro CAS : 3891-98-3), le squalane (ou 2,6,10,15,19,23-hexamethyltetracosane), le polyisobutène hydrogéné ou le polydécène hydrogéné ;
- Les mélanges d'alcanes comportant de 15 à 19 atomes de carbone, lesdits alcanes étant des alcanes linéaires, des alcanes ramifiés et des cyclo-alcanes, et plus particulièrement le mélange ($M_1$) qui comprend pour 100% de sa masse, une proportion massique en alcanes ramifiés supérieure ou égale à 90% et inférieure ou égale à 100% ; une proportion massique en alcanes linéaires supérieure ou égale à 0% et inférieure ou égale à 9%, et plus particulièrement inférieure à 5% et une proportion massique en cyclo-alcanes supérieure ou égale à 0% et inférieure ou égale à 1%, par exemple les mélanges commercialisés sous les noms Emogreen™L15 ou Emogreen™L19 ;

- Les éthers d'alcool gras de formule (II) :

$$Z_1\text{-O-}Z_2 \qquad (II),$$

dans laquelle $Z_1$ et $Z_2$ identiques ou différents, représentent un radical alkyle linéaire ou ramifié comportant de cinq à dix-huit atomes de carbone, par exemple les dioctyl éther, didécyl éther, didodécyl éther, dodécyl octyl éther, dihexadécyl éther, (1,3-diméthyl butyl) tétradécyl éther, (1,3-diméthyl butyl) hexadécyl éther, le bis(1,3-diméthyl butyl) éther ou le dihexyl éther.

- Les mono-esters d'acides gras et d'alcools de formule (III) :

$$R'_1\text{-(C=O)-O-}R'_2 \qquad (III),$$

dans laquelle $R'_1$-(C=O) représente un radical acyle, saturé ou insaturé, linéaire ou ramifié, comportant de huit à vingt-quatre atomes de carbone, et R'2 représente, indépendamment de $R'_1$, une chaîne hydrocarbonée saturée ou insaturée, linéaire ou ramifiée comportant de un à vingt-quatre atomes de carbone, par exemple les laurate de méthyle, laurate d'éthyle, laurate de propyle, laurate d'isopropyle, laurate de butyle, laurate de 2-butyle, laurate d'hexyle, cocoate de méthyle, cocoate d'éthyle, cocoate de propyle, cocoate d'isopropyle, cocoate de butyle, cocoate de 2-butyle, cocoate d'hexyle, myristate de méthyle, myristate d'éthyle, myristate de propyle, le myristate d'isopropyle, le myristate de butyle, le myristate de 2-butyle, le myristate d'hexyle, le myristate d'octyle, le palmitate de méthyle, le palmitate d'éthyle, le palmitate de propyle, le palmitate d'isopropyle, le palmitate de butyle, le palmitate de 2-butyle, le palmitate d'hexyle, le palmitate d'octyle , l'oléate de méthyle, l' oléate d'éthyle, l'oléate de propyle, l'oléate d'isopropyle, l'oléate de butyle, l'oléate de 2-butyle, l'oléate d'hexyle, l'oléate d'octyle, le stéarate de méthyle, le stéarate d'éthyle, le stéarate de propyle, le stéarate d'isopropyle, le stéarate de butyle, le stéarate de 2-butyle, le stéarate d'hexyle, le stéarate d'octyle, l'isostéarate de méthyle, l'isostéarate d'éthyle, l'isostéarate de propyle, l'isostéarate d'isopropyle, l'isostéarate de butyle, l'isostéarate de 2-butyle, l'isostéarate d'hexyle, l'isostéarate d'isostéaryle ;

- Les di-esters d'acides gras et de glycérol de formule (IV) et de formule (V) :

$$R'_3\text{-(C=O)-O-}CH_2\text{-CH(OH)-}CH_2\text{-O-(C=O)-}R'_4 \qquad (IV)$$

$$R'_5\text{-(C=O)-O-}CH_2\text{-CH[O-(C=O)-}R'_6\text{]-}CH_2\text{-OH} \qquad (V),$$

formules (VI) (VII) dans lesquelles $R'_3$-(C=O), $R'_4$-(C=O), $R'_5$-(C=O), $R'_6$-(C=O), identiques ou différents, représentent un groupement acyle, saturé ou insaturé, linéaire ou ramifié, comportant de huit à vingt-quatre atomes de carbone ;

- Les tri-esters d'acides gras et de glycérol de formule (VI) :

$$R'_7\text{-(C=O)-O-}CH_2\text{-CH[O-(C=O)-}R''_8\text{]-}CH_2\text{-O-(C=O)-}R''_9 \qquad (VI),$$

dans laquelle $R'_7$-(C=O), R's-(C=O) et $R'_9$-(C=O), identiques ou différents, représentent un groupement acyle, saturé ou insaturé, linéaire ou ramifié, comportant de huit à vingt-quatre atomes de carbone.

- Les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;
- Les huiles végétales éthoxylées.

De préférence, la composition selon l'invention comprend au moins une huile choisie parmi les éléments du groupe constitué par l'huile de ricin, les huiles de paraffine, le cocoyl caprylate/caprate, le myristate d'isopropyle, le capric caprylic triglycéride.

La phase grasse (A2) comprend optionnellement de la cire. Dans la formulation (F) à usage topique objet de la présente invention, on désigne par « cire » un composé et/ou un mélange de composés insoluble dans l'eau, et solide à 35°C

Une telle cire est plus particulièrement choisie parmi la cire d'abeille, la cire de carnauba, la cire de candelilla,

la cire d'ouricoury, la cire du Japon, la cire de fibre de liège, la cire de canne à sucre, les cires de paraffines, les cires de lignite, les cires microcristallines, la cire de lanoline; l'ozokérite; la cire de polyéthylène; les cires de silicone ; les cires végétales ; les alcools gras et les acides gras solides à température ambiante; les glycérides solides à température ambiante.

Dans la définition de la formulation (F) objet de la présente invention, on désigne par « compositions d'alkylpolyglycosides », une composition (A) représentée par la formule (VII) :

$$R_1\text{-O-}(G)_x\text{-H} \qquad (VII)$$

dans laquelle x représente un nombre décimal compris entre 1,05 et 5, G représente le reste d'un sucre réducteur, et $R_1$ représente un radical aliphatique hydrocarboné, saturé ou insaturé, linéaire ou ramifié, éventuellement substitué avec un ou plusieurs groupe hydroxyle, comportant de 12 à 36 atomes de carbone, ladite composition (A) consistant en un mélange de composés représentés par les formules ($VII_1$), ($VII_2$), ($VII_3$), ($VII_4$) et ($VII_5$) :

$$R_1\text{-O-}(G)_1\text{-H} \qquad (VII_1)$$

$$R_1\text{-O-}(G)_2\text{-H} \qquad (VII_2)$$

$$R_1\text{-O-}(G)_3\text{-H} \qquad (VII_3)$$

$$R_1\text{-O-}(G)_4\text{-H} \qquad (VII_4)$$

$$R_1\text{-O-}(G)_5\text{-H} \qquad (VII_5)$$

dans les proportions molaires respectives $a_1$, $a_2$, $a_3$, $a_4$ et $a_5$, telles que :

- La somme $a_1 + a_2 + a_3 + a_4 + a_5$ est égale à 1 et que
- La somme $a_1 + 2a_2 + 3a_3 + 4a_4 + 5a_5$ est égale à x.

Par radical aliphatique hydrocarboné, saturé ou insaturé, linéaire ou ramifié, comportant de 12 à 36 atomes de carbone, éventuellement substitué avec un ou plusieurs groupes hydroxyle, on désigne pour le radical $R_1$ dans la formule (VII) telle que définie ci-dessus :

- Les radicaux alkyle linéaires saturés, par exemple les radicaux n-dodécyle, n-tridécyle, n-tétradécyle, n-pentadécyle, n-hexadécyle, n-heptadécyle, n-octadécyle, n-nonadécyle, n-eicosyle, n-docosyle ;
- Les radicaux linéaires insaturés tels que les radicaux dodécènyle, tridécènyle, tétradécènyle, pentadécènyle, hexadécènyle, heptadécènyle, octadécènyle, nonadécènyle, eicosènyle, docosènyle, 4-dodécènyle, ou 5-dodécènyle ;
- Les radicaux aliphatiques saturés ou insaturés, linéaires ou ramifiés, comportant de 12 à 36 atomes de carbone substitués par un ou deux groupes hydroxy, tels que les radicaux hydroxydodécyle, hydroxytétradécyle, hydroxyhexadécyle, hydroxyoctadécyle, hydroxyeicosyle, hydroxydocosyle, par exemple le radical 12-hydroxy octadécyle.
- Les radicaux issus des isoalcanols de formule (1) :

$$(CH_3)(CH_3)CH\text{-}(CH_2)_r\text{-}CH_2\text{-OH} \qquad (1)$$

dans laquelle r représente un nombre entier compris entre 8 et 20, par exemple les radicaux isodécyle, isoundécyle, isododécyle, isotridécyle, isotétradécyle, isopentadécyle, isohexadécyle, isopentadécyle, isooctadécyle, isononadécyle, isoeicosyle ou isodocosyle ; Les radicaux alkyles ramifiés, issus des alcools de Guerbet, de formule (2) :

$$CH(C_sH_{2s+1})(CtH_{2t+1})\text{-}CH_2\text{-OH} \qquad (2)$$

dans laquelle t est un nombre entier compris entre 6 et 18, s est un nombre entier compris entre 4 et 18 et la somme s + t est supérieure ou égale à 10, et inférieure ou égale à 22, par exemple les radicaux 2-butyl octyle, 2-butyl décyle, 2-hexyl octyle, 2-hexyl décyle, 2-octyl décyle, 2-hexyl dodécyle, 2-octyl dodécyle, 2-décyl tétradécyle, 2-dodécyl hexadécyle, 2-tétradécyl octadécyle.

Selon un aspect particulier, dans la définition de la formule (VII) telle que définie ci-dessus, $R_1$ représente un radical aliphatique hydrocarboné, saturé ou insaturé, linéaire ou ramifié comportant de 12 à 24 atomes de carbone.

Par sucre réducteur, dans la définition de la formule (VII) telle que définie ci-dessus, on désigne les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990. La structure oligomérique $(G)_x$, peut se présenter sous toutes formes d'isomérie, qu'il s'agisse d'isomérie optique, d'isomérie géométrique ou d'isomérie de position ; elle peut aussi représenter un mélange d'isomères.

Dans la formule (VII) telle que définie ci-dessus, le groupe $R_1$-O- est lié à G par le carbone anomérique du reste saccharide, de manière à former une fonction acétal.

Selon un aspect particulier dans la définition de la formule (VII) telle que définie ci-dessus, G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose; et plus particulièrement G représente le reste d'un sucre réducteur choisi parmi les restes du glucose, du xylose et de l'arabinose.

Selon un aspect encore plus particulier, dans la définition de la formule (VII), x représente un nombre décimal supérieur ou égal à 1,05 et inférieur ou égal à 2,5, plus particulièrement supérieur ou égal à 1,05 et inférieur ou égal à 2,0, et encore plus particulièrement supérieur ou égal à 1,25 et inférieur ou égal à 2,0.

Selon un aspect encore plus particulier, dans la définition de la formule (VII) telle que définie ci-dessus, $R_1$ représente le radical choisi parmi au moins un des éléments du groupe constitué par les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle, 2-décyl tétradécyle ; G représente le reste d'un sucre réducteur choisi parmi les restes du glucose et du xylose et x représente un nombre décimal supérieur ou égal à 1,05 et inférieur ou égal à 2,5.

Selon un aspect encore plus particulier, la formulation (F) telle que définie précédemment, est caractérisée en ce que ledit système émulsionnant consiste en une composition (A) d'alkylpolyglycosides représentée par la formule (VII) :

$$R_1\text{-O-}(G)_x\text{-H} \quad \text{(VII)}$$

dans laquelle x représente un nombre décimal compris entre 1,05 et 2,5, G représente le reste du xylose, et $R_1$ représente le radical 2-octyl dodécyle, ladite composition (A) consistant en un mélange de composés représentés par les formules $(VII_1)$, $(VII_2)$, $(VII_3)$, $(VII_4)$ et $(VII_5)$ :

$$R_1\text{-O-}(G)_1\text{-H} \quad (VII_1)$$

$$R_1\text{-O-}(G)_2\text{-H} \quad (VII_2)$$

$$R_1\text{-O-}(G)_3\text{-H} \quad (VII_3)$$

$$R_1\text{-O-}(G)_4\text{-H} \quad (VII_4)$$

$$R_1\text{-O-}(G)s\text{-H} \quad (VII_5)$$

dans les proportions molaires respectives $a_1$, $a_2$, $a_3$, $a_4$ et $a_5$, telles que :

- La somme $a_1 + a_2 + a_3 + a_4 + a_5$ est égale à 1 et que
- La somme $a_1 + 2a_2 + 3a_3 + 4a_4 + 5a_5$ est égale à x.

Dans la définition de la composition (F) objet de la présente invention, on désigne par composition d'alkylpolyglycosides et d'alcools gras, une composition (B) comprenant pour 100% de sa masse :

- De 10% à 50% massique, plus particulièrement de 15% à 40% massique, et encore plus particulièrement de 20% à 30% massique, d'au moins une composition (A) représentée par la formule (VII) telle que définie précédemment,
- De 90% à 50% massique, plus particulièrement de 85% à 60% massique, et encore plus particulièrement de 80% à 70% massique, d'au moins un alcool gras de formule (VIII) :

$$R'_1\text{-OH} \quad \text{(VIII)},$$

dans laquelle $R'_1$, identique ou différent de $R_1$, représente un radical aliphatique hydrocarboné, saturé ou insaturé,

linéaire ou ramifié, éventuellement substitué avec un ou plusieurs groupe hydroxyle, comportant de 12 à 36 atomes de carbone.

Selon un aspect particulier, dans la définition de la formule (VII) représentant la composition (A) comprise dans la composition (B), $R_1$ représente le radical choisi parmi les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle, 2-décyl tétradécyle, G représente le reste d'un sucre réducteur choisi parmi les restes du glucose et du xylose et x représente un nombre décimal supérieur ou égal à 1,05 et inférieur ou égal à 2,5.

Selon un aspect plus particulier, dans la définition de la formule (VII) représentant la composition (A) comprise dans la composition (B), $R_1$ représente le radical 2-octyl dodécyle, G représente le reste du xylose et x représente un nombre décimal supérieur ou égal à 1,05 et inférieur ou égal à 2,5.

Selon un aspect plus particulier, dans la définition de l'alcool gras de formule (VIII) telle que définie ci-dessus, $R'_1$ représente un radical choisi parmi les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle ou 2-décyl tétradécyle, $R'_1$ représente tout particulièrement le radical 2-octyl dodécyle.

Selon un aspect encore plus particulier, l'invention a pour objet une formulation (F) telle que définie précédemment caractérisée en ce que ledit système émulsionnant consiste en une composition (B) comprenant pour 100% de sa masse :

- De 10% à 50% massique d'au moins une composition d'alkylpolyglycosides (A) représentée par la formule (VII) :

$$R_1\text{-O-(G)}_x\text{-H} \qquad \text{(VII)}$$

dans laquelle x représente un nombre décimal compris entre 1,05 et 2,5, G représente le reste du xylose et $R_1$ représente le radical 2-octyl dodécyle, ladite composition consistant en un mélange de composés représentés par les formules ($VII_1$), ($VII_2$), ($VII_3$), ($VII_4$) et ($VII_5$) :

$$R_1\text{-O-(G)}_1\text{-H} \qquad (VII_1)$$

$$R_1\text{-O-(G)}_2\text{-H} \qquad (VII_2)$$

$$R_1\text{-O-(G)}_3\text{-H} \qquad (VII_3)$$

$$R_1\text{-O-(G)}_4\text{-H} \qquad (VII_4)$$

$$R_1\text{-O-(G)}_5\text{-H} \qquad (VII_5)$$

dans les proportions molaires respectives $a_1$, $a_2$, $a_3$, $a_4$ et $a_5$, telles que :

- La somme $a_1 + a_2 + a_3 + a_4 + a_5$ est égale à 1 et que
- La somme $a_1 + 2a_2 + 3a_3 + 4a_4 + 5a_5$ est égale à x ; et
- De 90% à 50% massique d'au moins un alcool gras de formule (VIII) :

$$R'_1\text{-OH} \qquad \text{(VIII)},$$

dans laquelle $R'_1$ représente le radical 2-octyl dodécyle.

Dans la définition de la formulation (F) objet de la présente invention, on désigne par ester de polyglycérol, un composé de formule (IX) :

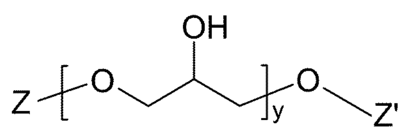

(IX)

dans laquelle Z représente un radical acyle de formule $R_2$-C(=O)-, dans laquelle $R_2$ représente un radical aliphatique

hydrocarboné, saturé ou insaturé, linéaire ou ramifié, comportant de 11 à 35 atomes de carbone et plus particulièrement un radical choisi parmi les radicaux dodécanoyle, tétradécanoyle, hexadécanoyle, octadécanoyle, eicosanoyle, docosanoyle, oléyle, linoléyle, linolénoyle ou isostéaryle, Z' représente le radical acyle de formule $R_2$-C(=O)-tel que défini ci-dessus, avec Z' identique ou différent de Z, ou l'atome d'hydrogène, et y représente un nombre entier supérieur ou égal à 2 et inférieur ou égal à 20.

Selon un aspect plus particulier, le composé de formule (IX) est choisi parmi les éléments du groupe constitué par l'oléate de décaglycérol, l'isostéarate de décaglycérol, le monolaurate de décaglycérol, le mono-linoléate de décaglycérol, le mono-myristate de décaglycérol.

Dans la définition de la formulation (F) objet de la présente invention, on désigne par esters de polyglycérols alcoxylés, un composé de formule (X) :

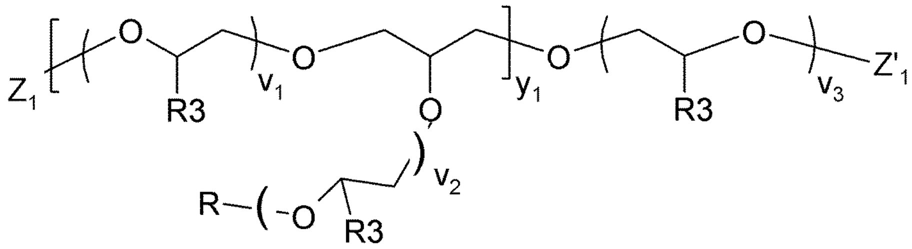

(X)

dans laquelle $Z_1$ représente un radical acyle de formule $R'_2$-C(=O)-, dans laquelle $R'_2$ représente un radical aliphatique hydrocarboné, saturé ou insaturé, linéaire ou ramifié, comportant de 11 à 35 atomes de carbone, et plus particulièrement un radical choisi parmi les radicaux radicaux dodécanoyle, tétradécanoyle, hexadécanoyle, octadécanoyle, eicosanoyle, docosanoyle, oléyle, linoléyle, linolénoyle ou isostéaryle, $Z_1$' représente le radical acyle de formule $R'_2$-C(=O)- tel que défini ci-dessus, avec $Z_1$' identique ou différent de $Z_1$, ou l'atome d'hydrogène, $R_3$ représente l'atome d'hydrogène, le radical méthyle, ou le radical éthyle, $y_1$ représente un nombre entier supérieur ou égal à 2 et inférieur ou égal à 20, $v_1$, $v_2$, $v_3$, identiques ou différents, représentent un nombre entier supérieur ou égal à 0 et inférieur ou égal à 50, et la somme $[(y_1.\ v_1) + (y_1.\ v_2) + v_3)]$ est un nombre entier supérieur ou égal à 1 et inférieur ou égal à 50.

Dans la définition de la composition (F) objet de la présente invention, on désigne par polyhydroxystéarates de polyglycols, un composé de formule (XI) :

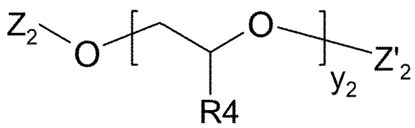

(XI)

dans laquelle $y_2$ représente un nombre entier supérieur ou égal à 2 et inférieur ou égal à 50, $R_4$ représente l'atome d'hydrogène, le radical méthyle, ou le radical éthyle, $Z_2$ représente un radical de formule (XII) :

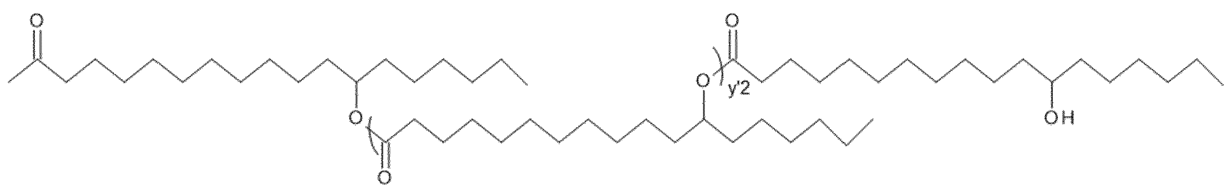

(XII),

dans laquelle $y'_2$ représente un nombre entier supérieur ou égal à 0 et inférieur ou égal à 10, plus particulièrement supérieur ou égal à 1 et inférieur ou égal à 10 et $Z'_2$ représente un radical de formule (XII) telle que définie ci-dessus, avec $Z_2$' identique ou différent de $Z_2$, ou l'atome d'hydrogène.

Dans la définition de la formulation (F) objet de la présente invention, on désigne par polyhydroxystéarate de polyglycérol, un composé représenté par la formule (XIII) :

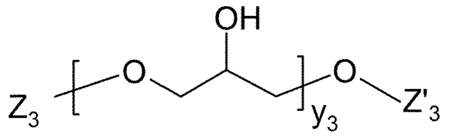

(XIII)

dans laquelle $Z_3$ représente un radical de formule (XII) telle que définie ci-dessus, $Z'_3$ représente un radical de formule (XII) telle que définie ci-dessus, avec $Z_3'$ identique ou différent de $Z_3$, ou l'atome d'hydrogène, $y_3$ représente un nombre entier supérieur ou égal à 2 et inférieur ou égal à 20.
Dans la définition de la formulation (F) objet de la présente invention, on désigne par polyhydroxystéarate de polyglycérol alcoxylé, un composé représenté par la formule (XIV) :

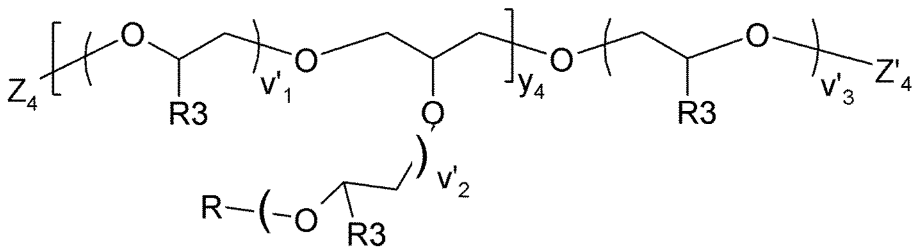

(XIV),

dans laquelle $Z_4$ représente un radical de formule (XII) telle que définie ci-dessus, $Z'_4$ représente un radical de formule (XII) telle que définie ci-dessus, avec $Z_4'$ identique ou différent de $Z_4$, ou l'atome d'hydrogène, $y_4$ représente un nombre entier supérieur ou égal à 2 et inférieur ou égal à 20, $v'_1$, $v'_2$, $v'_3$, identiques ou différents, représentent un nombre entier supérieur ou égal à 0 et inférieur ou égal à 50, et la somme $[(y_4. v'_1) + (y_4. v'_2) + v'_3)]$ est un nombre entier supérieur ou égal à 1 et inférieur ou égal à 50.

Selon un autre aspect particulier, l'invention a pour objet une formulation (F) telle que définie précédemment, caractérisée en ce que ledit système émulsionnant (S) consiste en une composition (D) comprenant pour 100% de sa masse :
De 15% à 25% massique d'au moins une composition (A) représentée par la formule (VII) :

$R1\text{-}O\text{-}(G)_x\text{-}H$ (VII)

dans laquelle x représente un nombre décimal compris entre 1,05 et 2,5, G représente le reste du xylose et $R_1$ représente le radical 2-octyl dodécyle, ladite composition (A) consistant en un mélange de composés représentés par les formules ($VII_1$), ($VII_2$), ($VII_3$), ($VII_4$) et ($VII_5$) :

$R_1\text{-}O\text{-}(G)_1\text{-}H$ ($VII_1$)

$R_1\text{-}O\text{-}(G)_2\text{-}H$ ($VII_2$)

$R_1\text{-}O\text{-}(G)_3\text{-}H$ ($VII_3$)

$R_1\text{-}O\text{-}(G)_4\text{-}H$ ($VII_4$)

$R_1\text{-}O\text{-}(G)_5\text{-}H$ ($VII_5$)

dans les proportions molaires respectives $a_1$, $a_2$, $a_3$, $a_4$ et $a_5$, telles que :

- La somme $a_1 + a_2 + a_3 + a_4 + a_5$ est égale à 1 et que
- La somme $a_1 + 2a_2 + 3a_3 + 4a_4 + 5a_5$ est égale à x ;

De 55% à 65% massique d'au moins un alcool gras de formule (VIII) :

$R'_1\text{-}OH$ (VIII),

dans laquelle $R'_1$ représente le radical 2-octyl dodécyle ;

De 10% à 30% massique d'au moins un polyhydroxystéarates de polyglycols représenté par la formule (XI) :

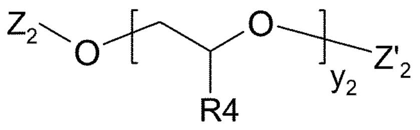

(XI)

dans laquelle $y_2$ représente un nombre entier supérieur ou égal à 2 et inférieur ou égal à 50, $R_4$ représente l'atome d'hydrogène, le radical méthyle ou le radical éthyle, $Z_2$ représente un radical de formule (XII) :

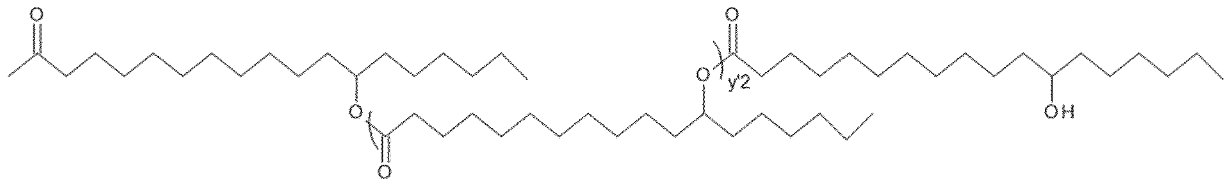

(XII),

dans laquelle $y'_2$ représente un nombre entier supérieur ou égal à 0 et inférieur ou égal à 10, plus particulièrement supérieur ou égal à 1 et inférieur ou égal à 10, $Z'_2$ représente un radical de formule (XII) telle que définie ci-dessus, avec $Z_2$' identique ou différent de $Z_2$, ou l'atome d'hydrogène.

Selon un autre aspect particulier, l'invention a pour objet une formulation (F) telle que définie précédemment caractérisée en ce que sa viscosité dynamique de ladite formulation (F), mesurée à une température de 25°C et à l'aide d'un viscosimètre Brookfield de type LVT à une vitesse de 6 tours/minute, est supérieure ou égale à 500 mPa.s et inférieure ou égale à 40 000 mPa.s.

La présente invention a également pour objet une composition à usage topique (F') se présentant sous la forme d'une émulsion huile-dans-eau et comprenant pour 100% de sa masse :

- de 50% à 90% massique, de préférence de 60 à 90% massique et encore plus préférentiellement de 70 à 90% massique d'une phase aqueuse (A1) cosmétiquement acceptable, ladite phase aqueuse (A1) comprenant pour 100% de sa propre masse de 0,5% à 10% massique du Lyzat (Ly) tel que défini précédemment.
- de 10% à 50% massique, de préférence de 10% à 40% massique et encore plus préférentiellement de 10% à 30% d'une phase grasse (G1) comprenant pour 100% de sa propre masse :
    - De 0,5% à 20% massique, de préférence de 1% à 15% massique d'au moins un agent tensioactif de type huile-dans-eau (S'1),
    - De 80% à 99,5% massique d'au moins une huile et/ou une cire.

Par « huile » présente dans la phase grasse ($G_1$) de la composition (F') se présentant sous la forme d'une émulsion de type huile-dans-eau telle que définie précédemment, on désigne au sens de la présente invention les substances chimiques ou les mélanges de substances chimiques insolubles dans l'eau, et se présentant sous un aspect liquide à une température de 25°C.

Par « cire » présente dans la phase grasse ($G_1$) de la composition (F') se présentant sous la forme d'une émulsion de type huile-dans-eau telle que définie précédemment, on désigne au sens de la présente invention les substances chimiques ou les mélanges de substances chimiques insolubles dans l'eau, et se présentant sous un aspect solide à une température de 45°C.

Par « agent tensioactif de type huile-dans-eau (S'1)» présent dans la phase grasse ($G_1$) de la composition (F') se présentant sous la forme d'une émulsion de type huile-dans-eau telle que définie précédemment, on désigne au sens de la présente invention, la substance chimique ou le mélange de substances chimiques qui permet de stabiliser les gouttelettes de ladite phase grasse ($G_1$) en dispersion dans la phase aqueuse continue ($A_1$).

Comme agent tensioactif de type huile-dans-eau (S'1) présent dans la phase grasse (G1) de l'émulsion (F') de type huile-dans-eau telle que définie précédemment, on peut citer par exemple:

- Des polysorbates résultant de la réaction d'éthoxylation entre un équivalent molaire d'esters de sorbitan et entre 5 et 20 équivalents molaires d'oxyde d'éthylène, et plus particulièrement entre un équivalent molaire de laurate de sorbitan, ou de palmitate de sorbitan, ou de stéarate de sorbitan, ou d'isostéarate de sorbitan, ou d'oléate de sorbitan,

et entre 5 et 20 équivalents molaires d'oxyde d'éthylène, plus particulièrement 5 ou 10 ou 12 ou 15 ou 20 équivalents molaires d'oxyde d'éthylène;

- Les produits résultant de la réaction d'éthoxylation entre un équivalent molaire d'un acide gras, comme par exemple l'acide palmitique, l'acide myristique, l'acide laurique, l'acide stéarique, l'acide isostéarique, l'acide oléique, et entre 5 à 40 équivalents molaires d'oxyde d'éthylène;
- Les produits résultant de la réaction d'estérification entre un acide gras, comme par exemple l'acide palmitique, l'acide myristique, l'acide laurique, l'acide stéarique, l'acide isostéarique, l'acide oléique, l'acide arachidique, l'acide béhénique et entre 4 à 20 équivalents molaires, plus particulièrement entre 3 à 10 équivalents molaires de glycérol.

Par "phase grasse (G1)", on désigne au sens de la présente invention, un corps gras ou un mélange de corps gras insoluble dans l'eau et/ou dans les mélanges d'eau et de solvants polaires. Une telle « phase grasse » peut comprendre des huiles et/ou des cires telles que définies précédemment.

Parmi les éléments constitutifs de la phase grasse (G1) présente dans la formulation (F'), on peut citer les huiles constitutives de la phase grasse (A2) telles que décrites ci-dessus pour la préparation de la formulation (F), et les huiles de silicone comme les diméthylpolysiloxanes, les méthylphényl-polysiloxanes, les silicones modifiées par des amines, les silicones modifiées par des acides gras, les silicones modifiées par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Parmi les éléments constitutifs de la phase grasse (G1) présente dans la formulation (F'), on peut citer les cires constitutives de la phase grasse (A2) telles que décrites ci-dessus pour la préparation de la formulation (F).

Selon un aspect particulier, dans la formulation (F') objet de la présente invention, la phase grasse (G1) comprend en outre pour 100% de sa propre masse de 5% à 30% massique, plus particulièrement de 5% à 25% massique, et encore plus particulièrement de 10% à 25% massique d'au moins un agent de protection contre les rayonnements ultra-violets du soleil. Par agent de protection contre les rayonnements ultra-violets du soleil, on désigne notamment dans la définition de la composition (F') à usage topique se présentant sous la forme d'une émulsion de type huile-dans-eau et objet de la présente invention, les pigments, les filtres organiques solaires et les filtres inorganiques solaires.

Comme pigments utilisés comme agent de protection contre les rayonnements ultra-violets du soleil, il y a par exemple le dioxyde de titane, les oxydes de fer marrons, les oxydes de fer jaune, les oxydes de fer noir, ou les oxydes de fer rouges ou encore les pigments nacrés blancs ou colorés tels que les Mica-Titane.

Comme filtres organiques solaires utilisés comme agent de protection contre les rayonnements ultra-violets du soleil, il y a par exemple:

- Ceux de la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA;
- Ceux de la famille des dérivés de l'acide anthranilique comme l'homomenthyl-N-acétyl anthranilate;
- Ceux de la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle;
- Ceux de la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-$\alpha$-cyano-$\beta$-phényle, le cinnamate de 2-éthylhexyl-$\alpha$-cyano-$\beta$-phényle, le cinnamate de diparaméthoxy mono-2-éthylhexanoyl de glycéryle;
- Ceux de la famille des dérivés de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le 2-éthylhexyl 4'-phényl benzophénone-2-carboxylate, la 2-hydroxy 4-n-octyloxy benzophénone, la 4-hydroxy 3-carboxy benzophénone ; le 3-(4'-méthylbenzylidène) d,l-camphre, le 3-(benzylidène)-d,l-camphre, le benzalkonium méthosulfate camphre ; l'acide urocanique, l'urocanate d'éthyle ;
- -Ceux de la famille des dérivés de l'acide sulfonique comme l'acide 2-phényl benzimidazole-5-sulfonique et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy) 1,3,5-triazine, le 4,4-((6-(((1,1-diméthyléthyl) amino) carbonyl) phenyl) amino)-1,3,5-triazine-2,4-diyl diimino) bis-(2-éthylhexyl) ester de l'acide benzoïque, le 2-phényl-5-méthyl benzoxazole, le 2,2'-hydroxy-5-méthylphényl benzotriazole, le 2-(2'-hydroxy-5'-t-octylphényl) benzotriazole, le 2-(2'-hydroxy-5'-méthyphényl) benzotriazole; la dibenzazine; le dianisoylméthane, le 4-méthoxy-4"-t-butylbenzoylméthane ; la 5-(3,3-diméthyl-2-norbornylidène)-3-pentan-2-one ; le 2-(4-diethylamino 2-hydroxy benzoyl) benzoic acid hexyl ester, le 2,4-bis{[4-(2-ethylhexyloxy)-2-hydroxy] phenyl}-6-(4-methoxy phenyl) 1,3,5-triazine, le 2,4,6-tris[4-(2-ethylhexyloxycarbonyl) anilino]-1,3,5-triazine, le 2-ethylhexyl dimethoxybenzylidene dioxoimidazolidine propionate, la famille des dérivés du diphénylacrylate comme le 2-éthylhexyl-2-cyano-3,3-diphényl-2-propènoate, l'éthyl-2-cyano-3,3-diphényl-2-propènoate;

- Ceux de la famille des polysiloxanes comme le malonate de benzylidène siloxane. Comme filtres inorganiques solaires utilisés comme agent de protection contre les rayonnements ultra-violets du soleil, il y a par exemple: les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

L'agent de protection contre les rayonnements ultra-violets du soleil sera de préférence choisi parmi les éléments du groupe constitué par dioxyde de titane, la 2,4-dihydroxy benzophénone, le 2-(4-diethylamino-2-hydroxybenzoyl) benzoic acid hexyl ester, le 2,4-bis{[4-(2-ethylhexyloxy)-2-hydroxy] phenyl}-6-(4-methoxyphenyl)-1,3,5-triazine, le 2,4,6-tris[4-(2-ethylhexyloxy carbonyl) anilino]-1,3,5-triazine et le 2-ethylhexyl dimethoxy benzylidene dioxoimidazolidine propionate.

L'expression "à usage topique" utilisée dans la définition de la composition (E1) se présentant sous la forme d'une émulsion de type huile-dans-eau telle que définie ci-dessus, signifie que ladite composition est formulée pour permettre son application sur la peau, les cheveux, le cuir chevelu ou les muqueuses, qu'il s'agisse d'une application directe dans le cas d'une composition cosmétique, dermocosmétique, dermopharmaceutique ou pharmaceutique ou d'une application indirecte par exemple dans le cas d'un produit d'hygiène corporelle sous forme de lingette en textile ou en papier ou de produits sanitaires destinés à être en contact avec la peau ou les muqueuses.

L'expression "cosmétiquement acceptable" utilisée dans la définition de la phase aqueuse ($A_1$) de la composition (E1) se présentant sous la forme d'une émulsion de type huile-dans-eau, signifie, selon la directive du Conseil de la Communauté Economique Européenne N°76/768/CEE du 27 juillet 1976 modifiée par la directive N°93/35/CEE du 14 juin 1993, toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Les compositions (F) et (F') pourront être administrable par voie topique pour réduire ou éliminer l'inflammation de la peau et/ou du cuir chevelu.

Selon un aspect encore plus particulier, la présente invention concerne une composition (F) et une composition (F') administrable par voie topique pour éliminer ou réduire les signes inesthétiques et/ou symptomatiques liés à l'inflammation de la peau humaine sensible et/ou du cuir chevelu comme par exemple les rougeurs, les tiraillements, les démangeaisons, les picotements.

Notons que les picotements et/ou les fourmillements et/ou les démangeaisons et/ou les échauffements et/ou les rougeurs et/ou l'inconfort cutané et/ou les tiraillements accompagnent les pathologies cutanées comme l'urticaire, les dermatites eczémateuses, la rosacée, le psoriasis, l'herpès, les photodermatoses, la dermatite atopique, la dermatite de contact, le lichen, les prurigos, les maladies prurigineuses, les fibroses, les troubles de la maturation du collagène, la sclérodermie, l'eczéma.

**1) préparation d'un lysat selon l'invention (LY)**

Un lysat (LY) de cellules dédifférenciées de la plante *Helichrysum stoechas* est préparé par la mise en oeuvre du procédé précédemment décrit, et plus particulièrement par la mise en oeuvre :

- d'une étape a) de préparation de l'échantillon d'Helichrysum stoechas stérile, telle que décrite précédemment, en utilisant des feuilles d'Helichrysum stoechas découpées en fragments de quelques millimètres, puis stérilisée par passage successif :
- dans un bain d'éthanol à 70% et de Tween™ 80 (à hauteur 0.05%) pendant cinq minutes
- dans un bain d'eau de javel à 1% pendant cinq minutes.

Les feuilles sont ensuite rincées dans de l'eau distillée stérile à trois reprises successives.

- D'une étape b) de callogénèse, telle que décrite précédemment, avec un milieu de callogénèse dont la constitution est décrite dans le tableau 1 ci-dessus, avec une phase d'incubation d'une durée de trois semaines à une température de 20 à 25°C à la lumière.
- D'une étape c) de mise en suspension des cellules dédifférenciées, telle que décrite précédemment, avec un milieu

de culture dont la composition est décrite dans le tableau 2 ci-dessus, pendant une durée de 14 à 30 jours, à une température de 20 à 25°C, à la lumière du jour, agitée par une agitation de type orbitale à une vitesse de 100 tours/minute.

- D'une étape d) de sélection d'une suspension fine telle que décrite précédemment, avec un prélèvement de 1/6 à 1/4 de culture obtenue à l'étape c), additionnée d'un volume de milieu de suspension tel que décrit dans le tableau 2 et en un volume ajusté au volume d'inoculum disponible dans le contenant.
  La nouvelle suspension est mise en culture dans une enceinte climatique pour une durée de 14 jours, à une température de 20 à 25°C, à la lumière du jour, agitée par une agitation de type orbitale à une vitesse de 100 tours/minute.
- D'une étape e) de production de biomasse telle que décrite précédemment, avec un prélèvement de 1/6 à 1/4 du milieu obtenu à l'étape d) additionnée d'un volume de milieu de suspension tel que décrit dans le tableau 2, et en un volume ajusté au volume d'inoculum disponible dans le contenant.
  La nouvelle suspension est mise en culture dans une enceinte climatique pour une durée de 14 jours dans un bioréacteur à vagues de capacité comprise entre 5 Litres et 1000 Litres, à une température de 20 à 25°C°C, à la lumière du jour, agitée par une agitation de type orbitale à une vitesse de 25 tours/minute.
- D'une étape f) d'homogénéisation à haute pression du milieu obtenu à l'issu de l'étape e), par passage dans un homogénéisateur à haute pression de marque GEA, à une pression de 500 bars sur une passe à une température inférieure à 40°C.
- On obtient ainsi un lysat (LY) comprenant pour 100% de sa masse :
- - 90-98 % massique d'eau
- - 10-2 % massique de matière sèche

D'une étape g) de stabilisation du milieu obtenu à l'étape f), en mélangeant 60% massique du milieu obtenu à l'étape f) avec 0.8% massique d'un agent épaississant commercialisé sous le nom de marque Solagum™AX, avec 37.9 % massique de glycérol, 0.3% de sorbate de potassium, de 1% de benzoate de sodium, mené à une valeur de 5.3 du pH

On obtient ainsi une composition $C_A$ comprenant pour 100% de sa masse :

- 60 % massique de lysat (LY)
- 37,9 % massique de glycérol
- 0,8 % massique de Solagum™ AX
- 0,3 % massique de sorbate de potassium
- 1 % massique de benzoate de sodium.

2) Mise en oeuvre expérimentale des effets biologiques d'un lysat de cellules dédifférenciées de la plante ***Helichrysum stoechas* selon l'invention**

Comme déjà mentionné le lysat tel que défini précédemment et obtenu par la mise en oeuvre d'un procédé comprenant les étapes a) à f) telles que décrites ci-dessus, permet de résoudre l'inflammation des peaux humaines, plus particulièrement sèches, réactives et/ou sensibles et ainsi de maintenir un bon niveau d'hydratation contribuant à réduire les sensations d'inconfort/de tiraillements, permettant ainsi d'apaiser, de contribuer au confort des peaux humaines, plus particulièrement sèches, réactives et/ou sensibles.

L'effet technique particulier du lysat tel que défini précédemment et obtenu par la mise en oeuvre d'un procédé comprenant les étapes a) à f) telles que décrites ci-dessus, a été mis en évidence sur plusieurs modèles d'étude in vitro et in vivo.

A- Effet apaisant

A.1) L'effet apaisant du Lysat (LY) tel que défini précédemment a été démontré à l'aide d'un modèle de co-culture de kératinocytes primaires humains avec des cellules denditritiques.

Les cellules dendritiques font parties des cellules du système immunitaire cutané, et elles sont impliquées dans la réponse inflammatoire précoce. Ce modèle vise à mimer les interactions qui se produisent naturellement dans la peau entre les kératinocytes et les cellules du système immunitaire, et il permet d'évaluer l'impact de principes actifs sur les processus de résolution de l'inflammation.

Brièvement, les kératinocytes humains normaux (NHEK) sont ensemencés dans des inserts et cultivés sans contact avec les cellules dendritiques (DC) ensemencées dans le fond des puits. Les cellules sont pré-traitées avec la composition $C_A$ ou la glycérine. Après 20 heures d'incubation, le stimulus inflammatoire (mélange de phorbol 12-myristate 13-acetate (« PMA »)et d'ionophore de calcium A23187), les acides gras polyinsaturés (PUFA, précurseurs lipidiques

des médiateurs de l'inflammation, les eicosanoïdes) et les composés (composition $C_A$ ou glycérol) ont été à nouveau ajoutés et incubés pendant 1 heure. Ensuite, les surnageants de culture ont été recueillis pour l'analyse de médiateurs lipidiques.

Les expérimentations ont été réalisées en trois réplicats.

Pour chaque surnageant de culture, on analyse :

- la quantité de 5-HETE (5-HydroxyEicosaTetraEnoic acid), médiateur pro-inflammatoire
- la quantité de LTB4 (leukotriene B4), médiateur pro-inflammatoire
- la quantité de 12-HETE (12-HydroxyEicosaTetraEnoic acid), médiateur pro-résolutif
- la quantité de 15-HETE (15-HydroxyEicosaTetraEnoic acid), médiateur pro-résolutif
- la quantité de 17-HDOHE ((+/-)-17-Hydroxy-4Z,7Z,10Z,13Z,15E,19Z-docosahexaenoic acid), médiateur pro-résolutif.

Les quantités de 5-HETE, LTB4, 12-HETE, 15-HETE, 17-HDoHE sont mesurées par la mise en oeuvre d'une méthode de LC-MS/MS utilisant un système UPLC (Agilent LC1290 Infinity) couplé à un spectromètre de masse Agilent 6490 triple quadripôle (Agilent Technologies) équipé d'une ionisation par électro-pulvérisation fonctionnant en mode négatif, et sont exprimées en picogrammes par puits de culture (pg/puits).

Les valeurs obtenues ont été exprimées en moyenne +/- sem [standard error of the mean ou erreur type de la moyenne = écart-type / racine carrée (nombre de valeurs)].

Pour chaque condition, les pourcentages d'activation ont été calculés comme suit :

% activation = [moyenne (condition)] / [moyenne (cellules stimulées)] x 100 – 100.

*cellules stimulées : cellules traitées PMA/A23187/PUFA dans le cas où la condition étudiée est le traitement avec la glycérine.*

*cellules stimulées : cellules traitées PMA/A23187/PUFA + DMSO 0,1% dans le cas où la condition étudiée est le traitement avec la composition $C_A$.*

L'analyse statistique a été réalisée par un test de Student et un seuil de significativité a été fixé à 5%, en comparant les conditions deux à deux. Une différence entre l'efficacité de deux produits a été considérée :

- Significative si $p < 0,05$ ;
- Dite « à la limite de significativité » si $0,05 \leq p < 0,1$ ;
- Et non significative si $p > 0,1$.

Les résultats sont présentés dans le tableau 4 ci-après :

[Table 3]

| | 5-HETE (pg/puits) Moyenne +/- SEM | % activation | LTB4 (pg/puits) Moyenne +/- SEM | % activation |
|---|---|---|---|---|
| cellules non traitées (contrôle) | 3,1 | - | 0 | - |
| PMA/A23187/PUFA | 472 +/- 26 | - | 213 +/- 8 | - |
| Glycérine (0,05%) | 1152 +/- 201 | 144%* | 400 +/- 38 | 88%* |
| PMA/A23187/PUFA+ DMSO 0,1% | 285 +/- 14 | - | 108 +/- 12 | - |
| Composition $C_A$ (0,1%) | 203 +/- 15 | -29%* | 83 +/- 4 | -24% |

| | 12-HETE (pg/puits) Moyenne +/- SEM | % activation | 15-HETE (pg/puits) Moyenne +/- SEM | % activation | 17-HDOHE (pg/puits) Moyenne +/- SEM | % activation |
|---|---|---|---|---|---|---|
| cellules non traitées (contrôle) | 0 | - | 40,5 | - | 17,3 | - |
| PMA/A23187/PUFA | 9 +/- 2 | - | 168 +/- 14 | - | 14768 +/- 464 | - |
| Glycérine (0,01%) | 15 +/- 1 | 58%$^{LS}$ | 273 +/- 26 | 63%* | 16654 +/- 1409 | +13% |
| PMA/A23187/PUFA + DMSO 0,1% | 7 +/- 2 | - | 103 +/- 15 | - | 10183 +/- 478 | - |
| Composition $C_A$ (0,01%) | 10 +/- 0 | 41%* | 152 +/- 13 | 48% | 12837 +/- 125 | 26%** |

*détermination de l'activation de la sécrétion de médiateurs pro- et anti-inflammatoires suite à la stimulation avec PMAIA23187IPUFA. LS 0,1<p<0,05, * p<0,05, ** p<0,01*

En conclusion, la composition $C_A$ est capable d'une part de diminuer la production de molécules pro-inflammatoires et d'autre part de stimuler la sécrétion de plusieurs médiateurs pro-résolutifs et ainsi de favoriser la résolution

de l'inflammation (production de lipoxines, résolvines de type D et protectines), permettant ainsi de réduire l'inflammation et favoriser le retour à l'homéostasie.

A.2) L'effet apaisant sur la peau altérée a également été montré à l'aide d'un modèle d'explants avec une barrière cutanée altérée. Cette altération de la fonction barrière est induite par un morceau de ruban adhésif appliqué sur la peau puis enlevé («tape-stripping ».

Cette méthode consiste à appliquer et à presser un morceau de ruban adhésif (de marque « D-Squame ») sur la surface de la peau, puis à le retirer. Les premières applications enlèvent une couche cellulaire complète de cornéocytes. La quantité de cornéocytes diminue sur le ruban adhésif au fur et à mesure des applications.

Les rubans adhésifs « D-Squame » sont appliqués sur les explants en veillant à ce qu'ils soient toujours réalisés au même endroit et de la même manière, avec une pression constante du pouce. Le ruban adhésif est ensuite retiré d'un mouvement fluide, rapide et unidirectionnel afin d'obtenir un retrait homogène des cornéocytes.

Ce modèle permet d'évaluer l'impact de formulations cosmétiques sur la physiologie des épidermes dont la fonction barrière est altérée.

Les expérimentations ont été réalisées en deux réplicats.

Les valeurs ont été exprimées en moyenne +/- sd [standard déviation = écart-type].

Pour chaque condition, les pourcentages de stimulation ou de protection ont été calculés comme suit :

% restauration = 100 x [moyenne(Composition CA) - moyenne (épidermes altérés)] / [moyenne(épidermes sains) - moyenne (épidermes altérés)].

La méthode utilisée est détaillée ci-après.

Les explants présentant une fonction barrière altérée (par la méthode de "tape-stripping"), et caractérisés par une perte insensible en eau supérieure à 35 g/m$^2$.h, sont traités une heure après l'altération de la fonction barrière, et tous les jours pendant cinq jours avec une formulation ($F_1$) comprenant 1% de la composition $C_A$, et dont la constitution massique est la suivante.

| | |
|---|---|
| Ingrédients | Teneur en pourcentage massique |
| Eau | QSP 100 % |
| Glycérol | 1% |
| Montanov™ L(1) | 1 |
| Simulsol™ 165(2) | 1 |
| Triglycerides C8-C10<br>Caprylic/Capric Triglycerides | 3 |
| DUB™ DNPG(3)<br>Neopentyl glycol diheptanoate | 7 |
| Sepimax™ ZEN (4)<br>Polyacrylate Crosspolymer-6 | 1 |
| Sepinov™ EMT 10 (5)<br>Hydroxyethyl Acrylate/Sodium Acryloyldimethyl Taurate Copolymer | 0,5 |
| Euxyl™ PE 9010 (6)<br>Phenoxylethanol - Ethylhexylglycerin | 1 |
| Sensiva™ PA40 (7)<br>3-Phenylpropan-1-ol -Octane-1,2-diol - Propane-1,3-diol | 0,5 |
| Composition $C_A$ | 1 |

(1) Montanov™L (nom INCI : C14-22 Alcohols & C12-20 Alkyl Glucoside) est une composition émulsionnante, destinée à stabiliser des émulsions, et utilisée pour la préparation de formulations cosmétiques.

(2) Simulsol™165 (nom INCI : PEG-100 Stéarate and Glyceryl Stéarate) est une composition émulsionnante, destinée à stabiliser des émulsions, notamment des émulsions huile-dans-eau, et utilisée pour la préparation de formulations cosmétiques.

(3) DUB™ DNPG (nom INCI : Neopentyl glycol diheptanoate" est un ester utilisé comme phase grasse et/ou agent émollient pour la préparation de formulations cosmétiques.

(4) Sepimax™ ZEN (nom INCI : Polyacrylate Crosspolymer-6) est un polyélectrolyte anionique réticulé utilisé comme agent épaississant et/ou émulsionnant et/ou stabilisant pour la préparation de formulations cosmétiques.

(5) Sepinov™ EMT 10 (nom INCI : Hydroxyethyl Acrylate/Sodium Acryloyldimethyl Taurate Copolymer" est un polyélectrolyte anionique réticulé utilisé comme agent épaississant et/ou émulsionnant et/ou stabilisant pour la préparation de formulations cosmétiques.

(6) Euxyl™ PE 9010 (nom INCI : Phenoxylethanol - Ethylhexylglycerin) est un agent conservateur utilisé pour la préparation de formulations cosmétiques.

(7) Sensiva™ PA40 (nom INCI : 3-Phenylpropan-1-ol -Octane-1,2-diol - Propane-1,3-diol) est un agent conservateur utilisé pour la préparation de formulations cosmétiques.

Les tissus sont digérés dans un mélange aqueux et organique. Une précipitation des protéines est réalisée. Plusieurs extractions successives sont ensuite réalisées pour purifier et concentrer l'échantillon.

Les extraits sont ensuite analysés à partir d'une méthode chromatographique LC/MS-MS permettant l'analyse quantitative des eicosanoïdes, médiateurs lipidiques de l'inflammation. L'analyse LC/MS-MS a été réalisée sur un système HPLC UltiMate 3000 (ThermoScientific) couplé à un spectromètre de masse MSQ Plus (ThermoScientific), équipé d'une ionisation par électro-pulvérisation fonctionnant en mode négatif.

Pour chaque extrait, on analyse :

- la quantité de 8-HETE (8-HydroxyEicosaTetraEnoic acid), médiateur pro-inflammatoire
- la quantité de PGE2 (Prostaglandine E2), médiateur pro-inflammatoire
- la quantité de de 15-HETE (15-HydroxyEicosaTetraEnoic acid), médiateur pro-résolutif Les quantités de 8-HETE, PGE2, de 15-HETE sont exprimées en unité arbitraire par milligramme de protéines (UA/mg de protéines).

Les résultats sont présentés dans le tableau 4 ci-après :

[Table 4]

| | 8-HETE (UA/mg de protéines) Moyenne +/- SD | % restauration | PGE2 (UA/mg de protéines) Moyenne +/- SD | % restauration |
|---|---|---|---|---|
| explants sains | 11,5 +/- 1,9 | - | 8,7 +/- 1,3 | - |
| explants altérés | 17,3 +/- 2,5 | - | 17,2 +/- 2,0 | - |
| Explants altérés + Formulation ($F_1$) à 1% de la Composition $C_A$ | 14,1+/-0,6 | 56% | 13,4 +/-1,1 | 45% |
| *restauration de la sécrétion de médiateurs pro-inflammatoires suite à l'altération de la fonction barrière par tape-stripping (pas de statistique - n=2)* | | | | |

| | 15-HETE (UA/mg de protéines) Moyenne +/- SD | % restauration |
|---|---|---|
| explants sains | 12,3 +/-1,5 | - |
| explants altérés | 5,9 +/- 1,0 | - |

(suite)

| | 15-HETE (UA/mg de protéines) Moyenne +/- SD | % restauration |
|---|---|---|
| Explants altérés + Formulation ($F_1$) à 1% de la Composition $C_A$ | 8,3 +/- 1,1 | 38% |

En conclusion, la composition $C_A$ est capable d'une part de diminuer la production de PGE2 médiateur pro-inflammatoire et d'autre part de stimuler la sécrétion de 15-HETE médiateur pro-résolutif et ainsi réduire l'inflammation et favoriser la résolution de l'inflammation et ainsi le retour à l'homéostasie.

En conclusion, les essais expérimentaux ont montré que la composition $C_A$ selon l'invention, comprenant le lysat (Ly) de cellules dédifférenciées de la plante Helichrysum stoechas, est capable de réduire les sensations d'inconfort associées aux peaux sèches et/ousensibles et/ou réactives, plus particulièrement de réduire les sensations de démangeaisons, de picotements, de brûlures, de douleurs, et de fourmillement, en stimulant la synthèse de médiateurs pro-résolutifs,

## B) Formulations

Dans les formules suivantes, les pourcentages sont exprimés en poids de la formulation.

| **B.1** | **Fluide démaquillant visage** |
|---|---|
| Formule | |
| Composition ($C_A$) | 10,00% |
| Méthyl paraben | 0,15% |
| Phenoxyethanol | 0,80% |
| SEPICALM™ S | 1,00% |
| Parfum/Fragrance | 0,10% |
| Eau | qs. 100,00% |

Mode opératoire : Mélanger les différents ingrédients dans l'eau sous agitation magnétique dans l'ordre indiqué, et ajuster le pH aux alentours de 7.

| **B.2** | **Shampoing cheveux et corps pour enfants** | |
|---|---|---|
| Formule | | |
| A | Composition ($C_A$) | 15,00% |
| | PROTEOL™APL | 5,00% |
| | SEPICIDE™HB | 0,50% |
| | Parfum/Fragrance | 0,10% |
| B | Eau | 20,00% |
| | CAPIGEL™98 | 3,50% |
| C | Eau | Q.S. 100,00% |
| | SEPICIDE™CI | 0,30% |
| | Colorant | Q.S |
| | Soude | Q.S. pH = 7,2 |

Mode opératoire : Mélanger la composition ($C_A$) avec le PROTEOL™APL, et le SEPICIDE™HB (Phase A). Diluer le CAPIGEL™98 dans une partie de l'eau et l'ajouter à la phase A précédemment obtenue (Phase B). Ajouter le reste d'eau à la phase B, puis le SEPICIDE™CI et le colorant. Ajuster le pH du mélange à 7,2 environ avec de la soude.

| B.3 | | Lingettes démaquillantes pour les yeux | |
|---|---|---|---|
| Formule | | | |
| A | Composition ($C_A$) | | 3,00% |
| B | | SEPICIDE™HB2 | 0,50% |
| C | | SEPICALM™ VG | 0,50% |
| | | Parfum/Fragrance | 0,05% |
| D | | Eau | Q.S. 100,00% |

Mode opératoire : Mélanger les ingrédients de la phase B ainsi que ceux de la phase C dans la phase A jusqu'à obtenir la limpidité de la solution. Ajouter la phase D.

| B.4 | | Gel moussant doux | |
|---|---|---|---|
| Formule | | | |
| A | Composition ($C_A$) | | 8,50% |
| | | PROTEOL™ APL | 3,00% |
| | | EUXYL ™ PE9010 | 1,00% |
| | | Parfum/Fragrance | 0,10% |
| B | | Eau | Q.S. 100,00% |
| | | Acide lactique | Q.S. pH = 6,0 |

Mode opératoire : Solubiliser le parfum et le conservateur EUXYL™ PE 9010 dans le mélange composé de la composition ($C_A$) et du PROTEOL™ APL (phase A). Ajouter l'eau et régler le pH à environ 6,0 avec de l'acide lactique.

| B.5 | Shampoing à usage fréquent | |
|---|---|---|
| Formule | | |
| A | Composition ($C_A$) | 12,80% |
| | PROTEOL™ OAT | 5,00% |
| | EUXYL ™ PE 9010 | 1,00% |
| | Parfum/Fragrance | 0,30% |
| | Eau Q.S. | 100,00% |
| B | MQNTALINE™C40 | 8,50% |
| | Acide lactique | Q.S. pH = 6,0 |

Mode opératoire : mélanger tous les ingrédients de la phase A et, après homogénéisation, ajouter la MONTALINE™C40 et ajuster le pH à environ 6,0 à l'aide de l'acide lactique.

| B.6 | Shampoing ultra-doux pour bébé | |
|---|---|---|
| Formule | | |
| A | Composition ($C_A$) | 10,00% |
| | AMISOFT™CS-11 | 4,00% |
| | Parfum/Fragrance | 0,10% |
| | SEPICIDE™HB | 0,30% |
| | SEPICIDE™CI | 0,20% |
| | Eau | Q.S. 100,00% |
| B | Eau | 20,00% |
| | CAPIGEL™ 98 | 3,50% |
| | Tromethamine | Q.S. pH = 7,2 |

Mode opératoire : Mélanger tous les ingrédients de la phase A dans l'ordre indiqué jusqu'à l'obtention d'une

phase A limpide. De façon séparée, ajouter le CAPIGEL™98 dans l'eau, puis ajouter cette phase B ainsi préparée dans la phase A et ajuster le pH à 7,2 à l'aide de la trométhamine.

| **B.7** | **Lait de toilette pour bébé** | |
|---|---|---|
| Formule | | |
| A | SIMULSOL™165 | 2,00% |
| | MONTANOV™202 | 1,00% |
| | LANOL™99 | 3,00% |
| | Dimethicone | 1,00% |
| | Isohexadecane | 3,00% |
| B | Eau | Q.S. 100,00% |
| C | SEPIPLUS™400 | 0,30% |
| D | Composition ($C_A$) | 6,35% |
| E | SEPICIDE™HB | 0,30% |
| | DMDM Hydantoin | 0,20% |
| | Parfum/Fragrance | 0,10% |

Mode opératoire : Faire chauffer séparément les phases A et B constituées par mélange des différents constituants. Ajouter la phase C dans la phase grasse chaude et réaliser l'émulsion en versant la phase aqueuse ; homogénéiser quelques minutes sous forte agitation (par l'intermédiaire d'une turbine rotor/stator). Puis ajouter la phase D dans l'émulsion chaude et refroidir l'émulsion sous agitation modérée jusqu'à retour à température ambiante. Ajouter la phase E à 40°C.

| **B.8** | **Lotion poudrée nettoyante pour peaux sensibles** | |
|---|---|---|
| Formule | | |
| A | LIPACIDE™C8G | 0,95% |
| | Méthyl paraben | 0,10% |
| | Ethyl paraben | 0,024% |
| | Propyl paraben | 0,0119% |
| | Butyl paraben | 0,024% |
| | Isobutyl paraben | 0,0119% |
| | Eau | 20,00% |
| | Disodium EDTA | 0,10% |
| | Triethanolamine | 1,38% |
| B | Composition ($C_A$) | 1,80% |
| | Parfum/Fragrance | 0,10% |
| C | SEPICALM™S | 0,28% |
| | Eau | Q.S. 100,00% |
| | Acide lactique | Q.S. pH = 5,2 |
| D | MICROPEARL™M310 | 5,00% |

Mode opératoire : Solubiliser les ingrédients de la phase A dans l'eau à 80°C. Solubiliser séparément le parfum dans la composition ($C_A$) pour préparer la phase B. Ajouter la phase A refroidie sur la phase B, puis introduire le SEPICALM™S et le complément d'eau. Vérifier le pH final et éventuellement l'ajuster à environ 5,2. Ajouter alors le MICROPEARL™ M310.

| **B.9** | **Gel douche enfants** | |
|---|---|---|
| Formule | | |
| A | Eau | 56,06% |

(suite)

| B.9 | Gel douche enfants | |
|---|---|---|
| Formule | | |
| | SEPIMAX™ Zen | 3,00% |
| | SEPIPLUS™ S | 0,80% |
| B | PROTEOL™ OAT | 20,80% |
| | ORAMIX™ NS 10 | 9,30% |
| | AMONYL™ 265 BA | 5,10% |
| C | Composition ($C_A$) | 2,00% |
| | Glycéryl Glucoside | 1,00% |
| | Phenoxyéthanol & Ethylhexyl Glycérine | 1,00% |
| | Parfum/Fragrance | 0,90% |
| | Colorant | 0,04% |

Mode opératoire : disperser le SEPIMAX™ZEN dans l'eau et agiter à l'aide d'un agitateur mécanique muni d'une défloculeuse, d'une contre-hélice et d'une pâle de type ancre, jusqu'à l'obtention d'un gel parfaitement lisse. Ajouter le SEPIPLUS™S puis agiter jusqu'à ce que le mélange soit homogène. Ajouter ensuite les ingrédients de la phase B, homogénéiser et ajouter individuellement les additifs de la phase C. Ajuster le pH à 6.0 - 6.5.

| B.10 | BB Crème | | |
|---|---|---|---|
| Formule | | | |
| A | EASYNOV™ | | 2,30% |
| | LANOL™ 99 | | 1,00% |
| | SEPIMAT™ H10W | | 1,00% |
| | Ethylhexyl methoxycinnamate | | 5,00% |
| B | Cyclométhicone | | 6,00% |
| | | Triethoxycaprylsilane & Alumina-silane & Titanium Oxide | 8,00% |
| | | Iron Oxide red & Triethoxycaprylsilane | 0,24% |
| | | Iron Oxide yellow & Triethoxycaprylsilane | 0,66% |
| | | Iron Oxide black & Triethoxycaprylsilane | 0,09% |
| | | Parfum/Fragrance | 0,10% |
| C | | Eau | qs 100% |
| | | SEPINOV™ EMT10 | 1,20% |
| D | Composition ($C_A$) | | 2,00% |
| | SEPITONIC™ M3 | | 1,00% |
| | | Phenoxyéthanol & Ethylhexyl Glycérine | 1,00% |

Mode opératoire : Préparer la phase B par mélange des différents ingrédients et homogénéiser à l'aide d'un mélangeur muni d'un système de rotor-stator à une vitesse de rotation de 4500 tours par minute, pendant une durée de 6 minutes. Préparer la phase C par addition du SEPINOV™EMT10 sur le mélange d'eau et de glycérol et homogénéiser à l'aide d'un mélangeur muni d'un système de rotor-stator à une vitesse de rotation de 4000 tours par minute pendant 4 minutes. Ajouter les phases A et B sur la phase C, et agiter le mélange résultant à l'aide d'un agitateur mécanique muni d'un pâle de type ancre, à une vitesse de 30 tours par minute pendant 2 minutes, puis à une vitesse de 50 tours par minute pendant 20 minutes. Ajouter un à un les composants de la phase D et agiter à une vitesse de 50 tours par minute pendant 25 minutes.

| B.11 | Spray Solaire haute Protection SPH supérieur à 30 | |
|---|---|---|
| Formule | | |
| A | MONTANOV™ L | 1,00% |
| | MONTANOV™ 82 | 1,00% |

(suite)

| B.11 | Spray Solaire haute Protection SPH supérieur à 30 | |
|---|---|---|
| Formule | | |
| | C12-15 Alkylbenzoate | 17,00% |
| | Dimethicone | 3,00% |
| | Octocrylène | 6,00% |
| | Ethylhexyl methoxycinnamate | 6,00% |
| | Bis-ethylhexyloxyphenol Méthoxypenyl Triazine | 3,00% |
| | Tocophérol | 0,05% |
| B | Eau | qs 100% |
| C | SIMULGEL™ INS 100 | 0,50% |
| | Cyclodiméthicone | 5,00% |
| D | Composition ($C_A$) | 3,00% |
| | Phenoxyéthanol & Ethylhexyl Glycérine | 1,00% |
| | Parfum/Fragrance | 0,20% |
| E | Methylene Bis-Benzotriazolyl Tetraméthylbutylphénol | 10,00% |
| | Acide citrique 25% | qs pH = 5 |

SEPICALM™S : Mélange de N-cocoyl aminoacides, de sarcosine, d'aspartate de potassium et d'aspartate de magnésium tel que décrit dans WO 98/09611.
PROTEOL™APL : Mélange de sels de sodium de N-cocoyl aminoacides, obtenus par acylation des acides aminés caractéristiques du jus de pomme ;
SEPICIDE™HB : Mélange de phénoxyéthanol, de méthylparaben, d'éthylparaben, de propylparaben et de butylparaben, est un agent conservateur.
CAPIGEL™98 : Copolymère d'acrylates ;
SEPICIDE™CI : Imidazoline urée, est un agent conservateur;
SEPICIDE™HB : Mélange de phénoxyéthanol, de méthylparaben, d'éthylparaben, de propylparaben, de butylparaben et d'isobutylparaben, est un agent conservateur ; SEPICALM™VG : Mélange de N-palmitoyl proline sous forme de sel de sodium et d'extrait de fleur de Nymphea Alba ;
EUXYL™PE9010 : Mélange de phénoxyéthanol et d'ethyl hexyl glycérine ;
PROTEOL™OAT : Mélange de N-lauryl aminoacides obtenus par hydrolyse totale de protéine d'avoine tel que décrit dans WO 94/26694 ;
MONTALINE™C40 : Sel de chlorure de Cocamidopropyl beta'inamide de Monoéthanolamine. AMISOFT™CS-11 : Sel de sodium de N-cocoyl glutamate ;
SIMULSOL™165 : Mélange de stéarate de PEG-100 et de stéarate de glycérol ; MONTANOV™202 (alcool arachydilique, alcool béhénique et arachidyl glucoside), est une composition auto-émulsionnable telle que celles décrites dans EP 0 977 626 ;
LANOL™99 : Isononoate d'isononyle ;
SEPIPLUS™400 : Latex inverse auto-inversible de polyacrylates dans le polyisobutene et comportant du polysorbate 20, tel que décrit dans WO2005/040230 ;
LIPACIDE™C8G : Capryloyl glycine commercialisé par la société SEPPIC ;
MICROPEARL™M310 : Polymère polyméthylméthacrylate réticulé se présentant sous forme de poudre et utilisé comme modificateur de texture ;
SEPIMAX™Zen (nom INCI : Polyacrylate Crosspolymer-6) : Polymère épaississant se présentant sous la forme d'une poudre ;
SEPIPLUS™S (nom INCI : Hydroxyethyl Acrylate / Sodium Acryloyldimethyl Taurate Copolymer & Polyisobutene & PEG-7 Trimethylolpropane Cononut Ether) : Latex inverse auto-inversible ; AMONYL™265 BA (nom INCI : Cocobétaïne) : Agent tensioactif amphotère moussant ; SEPINOV™EMT10 (nom INCI : Hydroxyethyl Acrylate / Sodium Acryloyldimethyl Taurate Copolymer) : Copolymère épaississant se présentant sous la forme d'une poudre ;
EASYNOV™ (nom INCI : Octyldodecanol and Octyldodecyl Xyloside and PEG-30 Dipolyhydroxystearate) : Agent émulsionnant à tendance lipophile ;
SEPIMAT™H10 FW (nom INCI : Methyl Méthacrylate Crosspolymer and Squalane) : Polymère utilisé comme agent de texture ;

SEPITONIC™M3 (nom INCI : Magnésium Aspartate and Zinc Gluconate and Copper Gluconate) : Mélange utilisé comme agent antiradicalaire et énergisant pour les cellules ;
MONTANOV™L (nom INCI : C14-22 Alcohols and C12-20 Alkylglucoside) : Agent émulsionnant ; MONTANOV™82 (nom INCI : Cetearyl Alcohol and Coco-glucoside) : Agent émulsionnant ; SIMULGEL™INS100 (nom INCI : Hydroxyethyl Acrylate/Sodium Acryloydimethyl Taurate Copolymer and isohexadecane and Polysorbate 60) : Agent épaississant polymérique ;

## Revendications

1. Lysat (Ly) de cellules dédifférenciées de la plante Helichrysum stoechas sous une forme adaptée à une administration par voie topique pour une utilisation pour éliminer ou réduire l'inflammation de la peau et/ou du cuir chevelu.

2. Lysat (Ly) pour une utilisation selon la revendication 1, **caractérisé en ce qu'**il est issu de l'homogénéisation haute pression de la culture de cellules dédifférenciées de la plante *Helichrysum stoechas.*

3. Composition à usage topique (C1) se présentant sous la forme d'un gel et comprenant pour 100% de sa masse :

   - De 95% à 99,5% massique du Lysat (Ly) tel que défini à la revendication 1 ou 2
   - De 0,5% à 5% massique d'au moins un agent épaississant et/ou gélifiant.

4. Composition (C1) selon la revendication 3, **caractérisée en ce que** les agents gélifiants et/ou épaississants sont choisis parmi les polysaccharides, la cellulose et les dérivés de la cellulose, les amidons, et les polymères de type polyélectrolytes, linéaires ou branchés ou réticulés.

5. Composition (C1) selon la revendication 3 ou 4 sous une forme adaptée à une administration par voie topique pour réduire ou éliminer l'inflammation de la peau et/ou du cuir chevelu.

6. Composition à usage topique (C'1) se présentant sous la forme d'un gel et comprenant pour 100% de sa masse :

   - De 50% à 80% massique du Lysat (Ly) tel que défini à la revendication 1 ou 2,
   - De 0,1% à 5% massique d'au moins un agent épaississant et/ou gélifiant, et
   - De 15% à 49,9% massique d'au moins un solvant.

7. Composition (C'1) selon la revendication 6, **caractérisé en ce que** le solvant est choisi parmi les éléments du groupe constitué par :

   - les composés de formule (Ia) :

   HO-[CH2-CH(OH)-CH2-O]n-H (Ia)

   dans laquelle n représente un nombre entier supérieur ou égal à un et inférieur ou égal à quinze, plus particulièrement supérieur ou égal à un et inférieur ou égal à 10, plus particulièrement supérieur ou égal à un et inférieur ou égal à 6, plus particulièrement supérieur ou égal à un et inférieur ou égal à 4, plus particulièrement égal à 1 ou 2 ou 3 ou 4 ;
   - les composés de formule (Ib) :

   Ra1-C(Rb1)(OH)-C(OH)(Rc1)(Rd1) (Ib),

   dans laquelle chacun des radicaux Ra1, Rb1, Rc1 et Rd1 représentent indépendamment les uns des autres, un atome d'hydrogène ou radical aliphatique saturé comportant de un à cinq atomes de carbone, soit par la formule (Ib1) :

   Ra1-C(Rb1)(OH)-[C(Re1)(Rf1)]t-C(OH)(Rc1)(Rd1) (Ib1),

   dans laquelle t est égal à un, deux ou trois, chacun des radicaux Ra1, Rb1, Rc1, Rd1, Re1 et Rf1 représentent indépendamment les uns des autres, un atome d'hydrogène ou radical aliphatique saturé comportant de un à cinq atomes de carbone, étant entendu que l'un au moins des radicaux Ra1 ou Rb1 et/ou l'un au moins des

radicaux Rc1 ou Rd1 ne représentent pas un atome d'hydrogène.

**8.** Composition (C'1) selon la revendication 6 ou 7, **caractérisée en ce que** le solvant est le glycérol.

**9.** Composition (C'1) selon l'une des revendications 6 à 8 sous une forme adaptée à une administration par voie topique pour réduire ou éliminer l'inflammation de la peau et/ou du cuir chevelu.

**10.** Composition à usage topique (C2) se présentant sous la forme d'une émulsion de type eau-dans-huile ou d'une émulsion de type huile-dans-eau, comprenant le lysat (Ly) tel que défini à la revendication 1 ou 2.

**11.** Composition à usage topique (F) se présentant sous la forme d'une émulsion eau-dans-huile et comprenant pour 100% de sa masse :

- de 60% à 90% massique de la composition à usage topique (C1) ou (C'1) telle que définie à l'une des revendications 3 à 9,
- de 10 % à 40 % massique d'une phase grasse ($A_2$) comprenant i) au moins une huile, et optionnellement au moins une cire et ii) un système émulsionnant comprenant au moins un agent tensioactif émulsionnant ($S_1$).

**12.** Composition (F) selon la revendication 11, **caractérisée en ce que** l'agent tensioactif émulsionnant ($S_1$) est choisi parmi les éléments du groupe constitué par les compositions d'alkylpolyglycosides, les compositions d'alkylpolyglycosides et d'alcools gras, les esters de polyglycérols, les esters de polyglycérols alcoxylés, les polyhydroxystéarates de polyglycols, les polyhydroxystéarates de polyglycérols, les polyhydroxystéarates de polyglycérols alcoxylés.

**13.** Composition à usage topique (F') se présentant sous la forme d'une émulsion huile-dans-eau et comprenant pour 100% de sa masse :

- de 50% à 90% massique d'une phase aqueuse (A1) cosmétiquement acceptable, ladite phase aqueuse (A1) comprenant pour 100% de sa propre masse de 0,5% à 10% massique du Lyzat (Ly) tel que défini à l'une des revendications 1 ou 2.
- de 10% à 50% massique d'une phase grasse (G1) comprenant pour 100% de sa propre masse :
  - De 0,5% à 20% massique d'au moins un agent tensioactif de type huile-dans-eau (S'1),
  - De 80% à 99,5% massique d'au moins une huile et/ou une cire.

**14.** Composition (C2), (F) ou (F') selon l'une des revendications 10 à 13 sous une forme adaptée à une administration par voie topique pour une utilisation pour réduire ou éliminer l'inflammation de la peau et/ou du cuir chevelu.

**15.** Composition (C2), (F) ou (F') selon l'une des revendications 10 à 13 pour une utilisation pour diminuer et/ou éliminer les picotements et/ou les fourmillements et/ou les démangeaisons et/ou les échauffements et/ou les rougeurs et/ou l'inconfort cutané et/ou les tiraillements de la peau provoqués par l'inflammation de la peau humaine et/ou le cuir chevelu.

## Patentansprüche

**1.** Lysat (Ly) von dedifferenzierten Zellen der Pflanze Helichrysum stoechas in einer für die topische Verabreichung geeigneten Form zur Verwendung zur Beseitigung oder Verringerung einer Entzündung der Haut und/oder der Kopfhaut.

**2.** Lysat (Ly) zur Verwendung nach Anspruch 1, **dadurch gekennzeichnet, dass** es aus der Hochdruckhomogenisierung der Kultur von dedifferenzierten Zellen der Pflanze *Helichrysum stoechas* stammt.

**3.** Zusammensetzung zur topischen Verwendung (C1), die in Form eines Gels vorliegt und auf 100 Gew.-% ihrer Masse

- 95 bis 99,5 Massen-% des Lysats (Ly) gemäß Anspruch 1 oder 2 und
- 0,5 bis 5 Massen-% mindestens eines Verdickungsmittels und/oder Gelierungsmittels umfasst.

**4.** Zusammensetzung (C1) nach Anspruch 3, **dadurch gekennzeichnet, dass** die Verdickungsmittel und/oder Gelierungsmittel aus Polysacchariden, Cellulose und Cellulosederivaten, Stärken und linearen oder verzweigten oder vernetzten Polymeren vom Polyelektrolyt-Typ ausgewählt sind.

**5.** Zusammensetzung (C1) nach Anspruch 3 oder 4 in einer für die topische Verabreichung geeigneten Form zur Verringerung oder Beseitigung einer Entzündung der Haut und/oder der Kopfhaut.

**6.** Zusammensetzung zur topischen Verwendung (C'1), die in Form eines Gels vorliegt und auf 100 Gew.-% ihrer Masse

- 50 bis 80 Massen-% des Lysats (Ly) gemäß Anspruch 1 oder 2,
- 0,1 bis 5 Massen-% mindestens eines Verdickungsmittels und/oder Gelierungsmittels und
- 15 bis 49,9 Massen-% mindestens eines Lösungsmittels umfasst.

**7.** Zusammensetzung (C'1) nach Anspruch 6, **dadurch gekennzeichnet, dass** das Lösungsmittel aus den Elementen der Gruppe bestehend aus

- Verbindungen der Formel (Ia):

$$HO\text{-}[CH_2\text{-}CH(OH)\text{-}CH_2\text{-}O]_n\text{-}H \qquad (Ia),$$

in der n für eine ganze Zahl größer oder gleich eins und kleiner oder gleich fünfzehn, spezieller größer oder gleich eins und kleiner oder gleich 10, spezieller größer oder gleich eins und kleiner oder gleich 6, spezieller größer oder gleich eins und kleiner oder gleich 4, spezieller gleich 1 oder 2 oder 3 oder 4, steht;
- Verbindung der Formel (Ib):

Ra1-C(Rb1)(OH)-C(OH)(Rc1)(Rd1) (Ib),

in der jeder der Reste Ra1, Rb1, Rc1 und Rd1 unabhängig voneinander für ein Wasserstoffatom oder einen gesättigten aliphatischen Rest mit eins bis fünf Kohlenstoffatomen steht, oder der Formel (Ib1):

Ra1-C(Rb1) (OH)-[C(Re1)(Rf1)]t-C(OH) (Rc1) (Rd1) (Ib1),

in der t gleich eins, zwei oder drei ist und jeder der Reste Ra1, Rb1, Rc1, Rd1, Re1 und Rf1 unabhängig voneinander für ein Wasserstoffatom oder einen gesättigten aliphatischen Rest mit eins bis fünf Kohlenstoffatomen steht, mit der Maßgabe, dass mindestens einer der Reste Ra1 und Rb1 und/oder mindestens einer der Reste Rc1 und Rd1 nicht für ein Wasserstoffatom steht; ausgewählt ist.

**8.** Zusammensetzung (C'1) nach Anspruch 6 oder 7, **dadurch gekennzeichnet, dass** es sich bei dem Lösungsmittel um Glycerin handelt.

**9.** Zusammensetzung (C'1) nach einem der Ansprüche 6 bis 8 in einer für die topische Verabreichung geeigneten Form zur Verringerung oder Beseitigung einer Entzündung der Haut und/oder der Kopfhaut.

**10.** Zusammensetzung zur topischen Verwendung (C2), die in Form einer Emulsion vom Wasser-in-Öl-Typ oder einer Emulsion vom Öl-in-Wasser-Typ vorliegt, umfassend das Lysat (Ly) gemäß Anspruch 1 oder 2.

**11.** Zusammensetzung zur topischen Verwendung (F), die in Form einer Wasser-in-Öl-Emulsion vorliegt und auf 100 Gew.-% ihrer Masse

- 60 bis 90 Massen-% der Zusammensetzung zur topischen Verwendung (C1) oder (C'1) gemäß einem der Ansprüche 3 bis 9 und
- 10 bis 40 Massen-% einer Fettphase ($A_2$), umfassend i) mindestens ein Öl und gegebenenfalls mindestens ein Wachs und ii) ein Emulgiersystem, das mindestens ein emulgierendes Tensid ($S_1$) umfasst,

umfasst.

**12.** Zusammensetzung (F) nach Anspruch 11, **dadurch gekennzeichnet, dass** das emulgierende Tensid ($S_1$) aus Elementen der Gruppe bestehend aus Zusammensetzungen von Alkylpolyglykosiden, Zusammensetzungen von Alkylpolyglykosiden und Fettalkoholen, Polyglycerinestern, alkoxylierten Polyglycerinestern, Polyglykolpolyhydroxystearaten, Polyglycerinpolyhydroxystearaten und alkoxylierten Polyglycerinpolyhydroxystearaten ausgewählt ist.

**13.** Zusammensetzung zur topischen Verwendung (F'), die in Form einer Wasser-in-Öl-Emulsion vorliegt und auf 100 Gew.-% ihrer Masse

- 50 bis 90 Massen-% einer kosmetisch unbedenklichen wässrigen Phase (A1), wobei die wässrige Phase (A1) auf 100 % ihrer eigenen Masse 0,5 bis 10 Massen-% Lysat (Ly) gemäß Anspruch 1 oder 2 umfasst,
- 10 bis 50 Massen-% einer Fettphase (G1), umfassend auf 100 % ihrer eigenen Masse

  • 0,5 bis 20 Massen-% mindestens eines Tensids vom Öl-in-Wasser-Typ (S'1),
  • 80 bis 99,5 Massen-% mindestens eines Öls und/oder eines Wachses,

umfasst.

**14.** Zusammensetzung (C2), (F) oder (F') nach einem der Ansprüche 10 bis 13 in einer für die topische Verabreichung geeigneten Form zur Verringerung oder Beseitigung einer Entzündung der Haut und/oder der Kopfhaut.

**15.** Zusammensetzung (C2), (F) oder (F') nach einem der Ansprüche 10 bis 13 zur Verwendung zur Verringerung und/oder Beseitigung von Prickeln und/oder Kribbeln und/oder Jucken und/oder Wärmegefühl und/oder Rötung und/oder Hautunbehagen und/oder Ziehen der Haut, das bzw. die durch die Entzündung der menschlichen Haut und/oder der Kopfhaut hervorgerufen werden.

**Claims**

**1.** Lysate (Ly) of dedifferentiated cells of the plant Helichrysum stoechas in a form suitable for topical administration, for use in eliminating or reducing inflammation of the skin and/or the scalp.

**2.** Lysate (Ly) for the use according to Claim 1, **characterized in that** it results from the high-pressure homogenization of the culture of dedifferentiated cells of the plant Helichrysum stoechas.

**3.** Composition for topical use (C1) which is in the form of a gel and which comprises, per 100% of its mass:

- from 95% to 99.5% by mass of the lysate (Ly) as defined in Claim 1 or 2,
- from 0.5% to 5% by mass of at least one thickening and/or gelling agent.

**4.** Composition (C1) according to Claim 3, **characterized in that** the gelling and/or thickening agents are chosen from polysaccharides, cellulose and cellulose derivatives, starches and linear or branched or crosslinked polymers of polyelectrolyte type.

**5.** Composition (C1) according to Claim 3 or 4 in a form suitable for topical administration for reducing or eliminating inflammation of the skin and/or the scalp.

**6.** Composition for topical use (C'1) which is in the form of a gel and which comprises, per 100% of its mass:

- from 50% to 80% by mass of the lysate (Ly) as defined in Claim 1 or 2,
- from 0.1% to 5% by mass of at least one thickening and/or gelling agent, and
- from 15% to 49.9% by mass of at least one solvent.

**7.** Composition (C'1) according to Claim 6, **characterized in that** the solvent is chosen from the elements of the group consisting of:

- the compounds of formula (Ia):

$$HO-[CH_2-CH(OH)-CH_2-O]_n-H \qquad (Ia)$$

in which n represents an integer greater than or equal to 1 and less than or equal to 15, more particularly greater than or equal to 1 and less than or equal to 10, more particularly greater than or equal to 1 and less than or

equal to 6, more particularly greater than or equal to 1 and less than or equal to 4, more particularly equal to 1 or 2 or 3 or 4;

- the compounds of formula (Ib):

Ra1-C(Rb1)(OH)-C(OH)(Rc1)(Rd1) (Ib),

in which each of the radicals Ra1, Rb1, Rc1 and Rd1 represent, independently of each other, a hydrogen atom or a saturated aliphatic radical comprising from 1 to 5 carbon atoms, or of formula (Ib1):

Ra1-C(Rb1)(OH)-$[C(Re1)(Rf1)]_t$-C(OH)(Rc1)(Rd1) (Ib1),

in which t is equal to 1, 2 or 3 and each of the radicals Ra1, Rb1, Rc1, Rd1, Re1 and Rf1 independently represent a hydrogen atom or a saturated aliphatic radical comprising from 1 to 5 carbon atoms, it being understood that at least one of the radicals Ra1 or Rb1 and/or at least one of the radicals Rc1 or Rd1 does not represent a hydrogen atom.

**8.** Composition (C'1) according to Claim 6 or 7, **characterized in that** the solvent is glycerol.

**9.** Composition (C'1) according to one of Claims 6 to 8 in a form suitable for topical administration for reducing or eliminating inflammation of the skin and/or the scalp.

**10.** Composition for topical use (C2) which is in the form of an emulsion of water-in-oil type or of an emulsion of oil-in-water type, comprising the lysate (Ly) as defined in Claim 1 or 2.

**11.** Composition for topical use (F) which is in the form of a water-in-oil emulsion and which comprises, per 100% of its mass:

- from 60% to 90% by mass of the composition for topical use (C1) or (C'1) as defined in one of Claims 3 to 9,
- from 10% to 40% by mass of a fatty phase ($A_2$) comprising i) at least one oil and optionally at least one wax and ii) an emulsifying system comprising at least one emulsifying surfactant ($S_1$).

**12.** Composition (F) according to Claim 11, **characterized in that** the emulsifying surfactant (S1) is chosen from the elements of the group consisting of alkylpolyglycoside compositions, compositions of alkylpolyglycosides and of fatty alcohols, polyglycerol esters, alkoxylated polyglycerol esters, polyglycol polyhydroxystearates, polyglycerol polyhydroxystearates and alkoxylated polyglycerol polyhydroxystearates.

**13.** Composition for topical use (F') which is in the form of an oil-in-water emulsion and which comprises, per 100% of its mass:

- from 50% to 90% by mass of a cosmetically acceptable aqueous phase (A1), said aqueous phase (A1) comprising, per 100% of its own mass, from 0.5% to 10% by mass of the lysate (Ly) as defined in either of Claims 1 and 2,
- from 10% to 50% by mass of a fatty phase (G1) comprising, per 100% of its own mass:
  - from 0.5% to 20% by mass of at least one surfactant of oil-in-water type (S'1),
  - from 80% to 99.5% by mass of at least one oil and/or one wax.

**14.** Composition (C2), (F) or (F') according to one of Claims 10 to 13 in a form suitable for topical administration, for use in reducing or eliminating inflammation of the skin and/or the scalp.

**15.** Composition (C2), (F) or (F') according to one of Claims 10 to 13, for use in reducing and/or eliminating stinging and/or tingling and/or itching and/or heating and/or red patches and/or skin discomfort and/or tautness of the skin caused by inflammation of human skin and/or scalp.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0234793 A2
- WO 9809611 A
- WO 9426694 A
- EP 0977626 A
- WO 2005040230 A